# EUROPEAN PATENT SPECIFICATION

(11) **EP 1 815 018 B1**
(45) Date of publication and mention of the grant of the patent: **15.07.2009**
(21) Application number: 05826422.7
(22) Date of filing: 21.11.2005
(51) Int. Cl.: C12Q 1/68

(54) **HUMAN OBESITY SUSCEPTIBILITY GENE ENCODING A MEMBER OF THE NEUREXIN FAMILY AND USES THEREOF**
FÜR EIN MITGLIED DER NEUREXINFAMILIE KODIERENDES HUMANES ADIPOSITAS-SUSZEPTIBILITÄTSSGEN UND DESSEN VERWENDUNG
GÈNE HUMAIN DE PRÉDISPOSITION À L'OBÉSITÉ CODANT POUR UN MEMBRE DE LA FAMILLE DES NEUREXINES ET UTILISATIONS DE CE GÈNE

(30) Priority: 23.11.2004 US 629946 P
(43) Date of publication of application: 08.08.2007
(73) Proprietor: Integragen, 91000 EVRY (FR)
(72) Inventor: PHILIPPI, Anne, F-77310 St. Fargeau Ponthierry (FR); ROUSSEAU, Francis, F-91300 Savigny sur Orge (FR); ROSCHMANN, Elke, D-89179 Beimerstetten (DE)
(74) Representative: Chajmowicz, Marion
(86) International application number: PCT/IB2005/003337
(87) International publication number: WO 2006/056839

(56) References cited:
- EP-A- 1 403 380
- US-A- 5 688 647
- SNYDER EE ET AL: "The human obesity gene map: the 2003 update" OBESITY RESEARCH, BATON ROUGE, LA,, US, vol. 12, no. 3, March 2004 (2004-03), pages 369-439, XP002348877 ISSN: 1071-7323
- DVORNYK V ET AL: "Current limitations of SNP data from public domain for studies of complex disorders: a test for ten candidate genes for obesity and osteoporosis" BMC GENETICS, BIOMED CENTRAL, GB, vol. 5, no. 4, February 2004 (2004-02), pages 1-15, XP002348876 ISSN: 1471-2156
- LEE Y S LOKE K Y: "The molecular pathogenesis of obesity: an unfinished jigsaw puzzle" ANNALS OF THE ACADEMY OF MEDICINE, SINGAPORE, ACADEMY OF MEDICINE, SG, vol. 29, no. 3, May 2000 (2000-05), pages 388-395, XP002953967 ISSN: 0304-4602
- EVANS D ET AL: "Association between polymorphisms in candidate genes and morbid obesity" INTERNATIONAL JOURNAL OF OBESITY, NEWMAN PUBLISHING, LONDON, GB, vol. 24, no. Suppl 1, May 2000 (2000-05), page S52,abstractno147, XP001207389 ISSN: 0307-0565
- BROOKS P ET AL: "GENOMIC HYBRID IDENTITY PROFILING (GENOMEHIP) OF CHROMOSOMAL REGIONS IDENTICAL BY DESCENT" AMERICAN JOURNAL OF HUMAN GENETICS, vol. 71, no. 4, SUPPL, October 2002 (2002-10), page 408, XP001109541 ISSN: 0002-9297

### SEQUENCE LISTING

<110> INTEGRAGEN
<120> HUMAN OBESITY SUSCEPTIBILITY GENE ENCODING A MEMBER OF THE NEUREXIN FAMILY AND USES THEREOF
<130> B0320US
<160> 27
<170> PatentIn version 3.1
<210> 1
   <211> 8107
   <212> DNA
   <213> Homo sapiens
<220>
   <221> CDS
   <222> (141)..(4136)
   <223>
<400> 1
   tgagggaaga agaggaagcg ggaggagctt ggcttcctcg cgtatttgag gacagcccat      60
   ctcccttcaa gaaccctacg gagagtcgga ctgcatctcc gcagcgagct cttggagcgc     120
   cgccggccgg gaggcgaagg atg cag gcg gct ccg cgc gcc ggc tgc ggg gca     173
                         Met Gln Ala Ala Pro Arg Ala Gly Cys Gly Ala
                         1               5                   10
   gcg ctc ctg ctg tgg att gtc agc agc tgc ctc tgc aga gcc tgg acg      221
   Ala Leu Leu Leu Trp Ile Val Ser Ser Cys Leu Cys Arg Ala Trp Thr
               15                  20                  25
   gct ccc tcc acg tcc caa aaa tgt gat gag cca ctt gtc tct gga ctc      269
   Ala Pro Ser Thr Ser Gln Lys Cys Asp Glu Pro Leu Val Ser Gly Leu
           30                  35                  40
   ccc cat gtg gct ttc agc agc tcc tcc tcc atc tct ggt agc tat tct      317
   Pro His Val Ala Phe Ser Ser Ser Ser Ser Ile Ser Gly Ser Tyr Ser
       45                  50                  55
   ccc ggc tat gcc aag ata aac aag aga gga ggt gct ggg gga tgg tct      365
   Pro Gly Tyr Ala Lys Ile Asn Lys Arg Gly Gly Ala Gly Gly Trp Ser
   60                  65                  70                  75
   cca tca gac agc gac cat tat caa tgg ctt cag gtt gac ttt ggc aat      413
   Pro Ser Asp Ser Asp His Tyr Gln Trp Leu Gln Val Asp Phe Gly Asn
                   80                  85                  90
   cgg aag cag atc agt gcc att gca acc caa gga agg tat agc agc tca      461
   Arg Lys Gln Ile Ser Ala Ile Ala Thr Gln Gly Arg Tyr Ser Ser Ser
               95                  100                 105
   gat tgg gtg acc caa tac cgg atg ctc tac agc gac aca ggg aga aac      509
   Asp Trp Val Thr Gln Tyr Arg Met Leu Tyr Ser Asp Thr Gly Arg Asn
           110                 115                 120
   tgg aaa ccc tat cat caa gat ggg aat atc tgg gca ttt ccc gga aac      557
   Trp Lys Pro Tyr His Gln Asp Gly Asn Ile Trp Ala Phe Pro Gly Asn
       125                 130                 135
   att aac tct gac ggt gtg gtc cgg cac gaa tta cag cat ccg att att      605
   Ile Asn Ser Asp Gly Val Val Arg His Glu Leu Gln His Pro Ile Ile
   140                 145                 150                 155
   gcc cgc tat gtg cgc ata gtg cct ctg gat tgg aat gga gaa ggt cgc      653
   Ala Arg Tyr Val Arg Ile Val Pro Leu Asp Trp Asn Gly Glu Gly Arg
                   160                 165                 170
   att gga ctc aga att gaa gtt tat ggc tgt tct tac tgg gct gat gtt      701
   Ile Gly Leu Arg Ile Glu Val Tyr Gly Cys Ser Tyr Trp Ala Asp Val
               175                 180                 185
   atc aac ttt gat ggc cat gtt gta tta cca tat aga ttc aga aac aag      749
   Ile Asn Phe Asp Gly His Val Val Leu Pro Tyr Arg Phe Arg Asn Lys
           190                 195                 200
   aag atg aaa aca ctg aaa gat gtc att gcc ttg aac ttt aag acg tct      797
   Lys Met Lys Thr Leu Lys Asp Val Ile Ala Leu Asn Phe Lys Thr Ser
       205                 210                 215
   gaa agt gaa gga gta atc ctg cac gga gaa gga cag caa gga gat tac      845
   Glu Ser Glu Gly Val Ile Leu His Gly Glu Gly Gln Gln Gly Asp Tyr
   220                 225                 230                 235
   att acc ttg gaa ctg aaa aaa gcc aag ctg gtc ctc agt tta aac tta      893
   Ile Thr Leu Glu Leu Lys Lys Ala Lys Leu Val Leu Ser Leu Asn Leu
                   240                 245                 250
   gga agc aac cag ctt ggc ccc ata tat ggc cac aca tca gtg atg aca      941
   Gly Ser Asn Gln Leu Gly Pro Ile Tyr Gly His Thr Ser Val Met Thr
               255                 260                 265
   gga agt ttg ctg gat gac cac cac tgg cac tct gtg gtc att gag cgc      989
   Gly Ser Leu Leu Asp Asp His His Trp His Ser Val Val Ile Glu Arg
           270                 275                 280
   cag ggg cgg agc att aac ctc act ctg gac agg agc atg cag cac ttc     1037
   Gln Gly Arg Ser Ile Asn Leu Thr Leu Asp Arg Ser Met Gln His Phe
       285                 290                 295
   cgt acc aat gga gag ttt gac tac ctg gac ttg gac tat gag ata acc     1085
   Arg Thr Asn Gly Glu Phe Asp Tyr Leu Asp Leu Asp Tyr Glu Ile Thr
   300                 305                 310                 315
   ttt gga ggc atc cct ttc tct ggc aag ccc agc tcc agc agt aga aag     1133
   Phe Gly Gly Ile Pro Phe Ser Gly Lys Pro Ser Ser Ser Ser Arg Lys
                   320                 325                 330
   aat ttc aaa ggc tgc atg gaa agc atc aac tac aat ggc gtc aac att     1181
   Asn Phe Lys Gly Cys Met Glu Ser Ile Asn Tyr Asn Gly Val Asn Ile
               335                 340                 345
   act gat ctt gcc aga agg aag aaa tta gag ccc tca aat gtg gga aat     1229
   Thr Asp Leu Ala Arg Arg Lys Lys Leu Glu Pro Ser Asn Val Gly Asn
           350                 355                 360
   ttg agc ttt tct tgt gtg gaa ccc tat acg gtg cct gtc ttt ttc aac     1277
   Leu Ser Phe Ser Cys Val Glu Pro Tyr Thr Val Pro Val Phe Phe Asn
       365                 370                 375
   gct aca agt tac ctg gag gtg ccc gga cgg ctt aac cag gac ctg ttc      1325
   Ala Thr Ser Tyr Leu Glu Val Pro Gly Arg Leu Asn Gln Asp Leu Phe
   380                 385                 390                 395
   tca gtc agt ttc cag ttt agg aca tgg aac ccc aat ggt ctc ctg gtc      1373
   Ser Val Ser Phe Gln Phe Arg Thr Trp Asn Pro Asn Gly Leu Leu Val
                   400                 405                 410
   ttc agt cac ttt gcg gat aat ttg ggc aat gtg gag att gac ctc act      1421
   Phe Ser His Phe Ala Asp Asn Leu Gly Asn Val Glu Ile Asp Leu Thr
               415                 420                 425
   gaa agc aaa gtg ggt gtt cac atc aac atc aca cag acc aag atg agc      1469
   Glu Ser Lys Val Gly Val His Ile Asn Ile Thr Gln Thr Lys Met Ser
           430                 435                 440
   caa atc gat att tcc tca ggt tct ggg ttg aat gat gga cag tgg cac      1517
   Gln Ile Asp Ile Ser Ser Gly Ser Gly Leu Asn Asp Gly Gln Trp His
       445                 450                 455
   gag gtt cgc ttc cta gcc aag gaa aat ttt gct att ctc acc atc gat      1565
   Glu Val Arg Phe Leu Ala Lys Glu Asn Phe Ala Ile Leu Thr Ile Asp
   460                 465                 470                 475
   gga gat gaa gca tca gca gtt cga act aat agt ccc ctt caa gtt aaa      1613
   Gly Asp Glu Ala Ser Ala Val Arg Thr Asn Ser Pro Leu Gln Val Lys
                   480                 485                 490
   act ggc gag aag tac ttt ttt gga ggt ttt ctg aac cag atg aat aac      1661
   Thr Gly Glu Lys Tyr Phe Phe Gly Gly Phe Leu Asn Gln Met Asn Asn
               495                 500                 505
   tca agt cac tct gtc ctt cag cct tca ttc caa gga tgc atg cag ctc      1709
   Ser Ser His Ser Val Leu Gln Pro Ser Phe Gln Gly Cys Met Gln Leu
           510                 515                 520
   att caa gtg gac gat caa ctt gta aat tta tac gaa gtg gca caa agg      1757
   Ile Gln Val Asp Asp Gln Leu Val Asn Leu Tyr Glu Val Ala Gln Arg
       525                 530                 535
   aag ccg gga agt ttc gcg aat gtc agc att gac atg tgt gcg atc ata      1805
   Lys Pro Gly Ser Phe Ala Asn Val Ser Ile Asp Met Cys Ala Ile Ile
   540                 545                 550                 555
   gac aga tgt gtg ccc aat cac tgt gag cat ggt gga aag tgc tcg caa      1853
   Asp Arg Cys Val Pro Asn His Cys Glu His Gly Gly Lys Cys Ser Gln
                   560                 565                 570
   aca tgg gac agc ttc aaa tgc act tgt gat gag aca gga tac agt ggg      1901
   Thr Trp Asp Ser Phe Lys Cys Thr Cys Asp Glu Thr Gly Tyr Ser Gly
               575                 580                 585
   gcc acc tgc cac aac tct atc tac gag cct tcc tgt gaa gcc tac aaa      1949
   Ala Thr Cys His Asn Ser Ile Tyr Glu Pro Ser Cys Glu Ala Tyr Lys
           590                 595                 600
   cac cta gga cag aca tca aat tat tac tgg ata gat cct gat ggc agc      1997
   His Leu Gly Gln Thr Ser Asn Tyr Tyr Trp Ile Asp Pro Asp Gly Ser
   605 610 615
   gga cct ctg ggg cct ctg aaa gtt tac tgc aac atg aca gag gac aaa 2045
   Gly Pro Leu Gly Pro Leu Lys Val Tyr Cys Asn Met Thr Glu Asp Lys
   620 625 630 635
   gtg tgg acc ata gtg tct cat gac ttg cag atg cag acg cct gtg gtc 2093
   Val Trp Thr Ile Val Ser His Asp Leu Gln Met Gln Thr Pro Val Val
   640 645 650
   ggc tac aac cca gaa aaa tac tca gtg aca cag ctc gtt tac agc gcc 2141
   Gly Tyr Asn Pro Glu Lys Tyr Ser Val Thr Gln Leu Val Tyr Ser Ala
   655 660 665
   tcc atg gac cag ata agt gcc atc act gac agt gcc gag tac tgc gag 2189
   Ser Met Asp Gln Ile Ser Ala Ile Thr Asp Ser Ala Glu Tyr Cys Glu
   670 675 680
   cag tat gtc tcc tat ttc tgc aag atg tca aga ttg ttg aac acc cca 2237
   Gln Tyr Val Ser Tyr Phe Cys Lys Met Ser Arg Leu Leu Asn Thr Pro
   685 690 695
   gat gga agc cct tac act tgg tgg gtt ggc aaa gcc aac gag aag cac 2285
   Asp Gly Ser Pro Tyr Thr Trp Trp Val Gly Lys Ala Asn Glu Lys His
   700 705 710 715
   tac tac tgg gga ggc tct ggg cct gga atc cag aaa tgt gcc tgc ggc 2333
   Tyr Tyr Trp Gly Gly Ser Gly Pro Gly Ile Gln Lys Cys Ala Cys Gly
   720 725 730
   atc gaa cgc aac tgc aca gat ccc aag tac tac tgt aac tgc gac gcg 2381
   Ile Glu Arg Asn Cys Thr Asp Pro Lys Tyr Tyr Cys Asn Cys Asp Ala
   735 740 745
   gac tac aag caa tgg agg aag gat gct ggt ttc tta tca tac aaa gat 2429
   Asp Tyr Lys Gln Trp Arg Lys Asp Ala Gly Phe Leu Ser Tyr Lys Asp
   750 755 760
   cac ctg cca gtg agc caa gtg gtg gtt gga gat act gac cgt caa ggc 2477
   His Leu Pro Val Ser Gln Val Val Val Gly Asp Thr Asp Arg Gln Gly
   765 770 775
   tca gaa gcc aaa ttg agc gta ggt cct ctg cgc tgc caa gga gac agg 2525
   Ser Glu Ala Lys Leu Ser Val Gly Pro Leu Arg Cys Gln Gly Asp Arg
   780 785 790 795
   aat tat tgg aat gcc gcc tct ttc cca aac cca tcc tcc tac ctg cac 2573
   Asn Tyr Trp Asn Ala Ala Ser Phe Pro Asn Pro Ser Ser Tyr Leu His
   800 805 810
   ttc tct act ttc caa ggg gaa act agc gct gac att tct ttc tac ttc 2621
   Phe Ser Thr Phe Gln Gly Glu Thr Ser Ala Asp Ile Ser Phe Tyr Phe
   815 820 825
   aaa aca tta acc ccc tgg gga gtg ttt ctt gaa aat atg gga aag gaa 2669
   Lys Thr Leu Thr Pro Trp Gly Val Phe Leu Glu Asn Met Gly Lys Glu
   830 835 840
   gat ttc atc aag ctg gag ctg aag tct gcc aca gaa gtg tcc ttt tca 2717
   Asp Phe Ile Lys Leu Glu Leu Lys Ser Ala Thr Glu Val Ser Phe Ser
   845 850 855
   ttt gat gtg gga aat ggg cca gta gag att gta gtg agg tca cca acc      2765
   Phe Asp Val Gly Asn Gly Pro Val Glu Ile Val Val Arg Ser Pro Thr
   860                 865                 870                 875
   cct ctc aac gat gac cag tgg cac cgg gtc act gca gag agg aat gtc      2813
   Pro Leu Asn Asp Asp Gln Trp His Arg Val Thr Ala Glu Arg Asn Val
                   880                 885                 890
   aag cag gcc agc cta cag gtg gac cgg cta ccg cag cag atc cgc aag      2861
   Lys Gln Ala Ser Leu Gln Val Asp Arg Leu Pro Gln Gln Ile Arg Lys
               895                 900                 905
   gcc cca aca gaa ggc cac acc cgc ctg gag ctc tac agc cag tta ttt      2909
   Ala Pro Thr Glu Gly His Thr Arg Leu Glu Leu Tyr Ser Gln Leu Phe
           910                 915                 920
   gtg ggt ggt gct ggg ggc cag cag ggc ttc ctg ggc tgc atc cgc tcc      2957
   Val Gly Gly Ala Gly Gly Gln Gln Gly Phe Leu Gly Cys Ile Arg Ser
       925                 930                 935
   ttg agg atg aat ggg gtg aca ctt gac ctg gag gaa aga gca aag gtc      3005
   Leu Arg Met Asn Gly Val Thr Leu Asp Leu Glu Glu Arg Ala Lys Val
   940                 945                 950                 955
   aca tct ggg ttc ata tcc gga tgc tcg ggc cat tgc acc agc tat gga      3053
   Thr Ser Gly Phe Ile Ser Gly Cys Ser Gly His Cys Thr Ser Tyr Gly
                   960                 965                 970
   aca aac tgt gaa aat gga ggc aaa tgc cta gag aga tac cac ggt tac      3101
   Thr Asn Cys Glu Asn Gly Gly Lys Cys Leu Glu Arg Tyr His Gly Tyr
               975                 980                 985
   tcc tgc gat tgc tct aat act gca tat gat gga aca ttt  tgc aac aaa     3149
   Ser Cys Asp Cys Ser Asn Thr Ala Tyr Asp Gly Thr Phe  Cys Asn Lys
           990                 995                 1000
   gat gtt  ggt gca ttt ttt gaa  gaa ggg atg tgg cta  cga tat aac       3194
   Asp Val  Gly Ala Phe Phe Glu  Glu Gly Met Trp Leu  Arg Tyr Asn
       1005                 1010                 1015
   ttt cag  gca cca gca aca aat  gcc aga gac tcc agc  agc aga gta       3239
   Phe Gln  Ala Pro Ala Thr Asn  Ala Arg Asp Ser Ser  Ser Arg Val
       1020                 1025                 1030
   gac aac  gct ccc gac cag cag  aac tcc cac ccg gac  ctg gca cag       3284
   Asp Asn  Ala Pro Asp Gln Gln  Asn Ser His Pro Asp  Leu Ala Gln
       1035                 1040                 1045
   gag gag  atc cgc ttc agc ttc  agc acc acc aag gcg  ccc tgc att       3329
   Glu Glu  Ile Arg Phe Ser Phe  Ser Thr Thr Lys Ala  Pro Cys Ile
       1050                 1055                 1060
   ctc ctc  tac atc agc tcc ttc  acc aca gac ttc ttg  gca gtc ctc       3374
   Leu Leu  Tyr Ile Ser Ser Phe  Thr Thr Asp Phe Leu  Ala Val Leu
       1065                 1070                 1075
   gtc aaa  ccc act gga agc tta  cag att cga tac aac  ctg ggt ggc       3419
   Val Lys  Pro Thr Gly Ser Leu  Gln Ile Arg Tyr Asn  Leu Gly Gly
       1080                 1085                 1090
   acc cga  gag cca tac aat att  gac gta gac cac agg  aac atg gcc      3464
   Thr Arg  Glu Pro Tyr Asn Ile  Asp Val Asp His Arg  Asn Met Ala
       1095                 1100                 1105
   aat gga  cag ccc cac agt gtc  aac atc acc cgc cac  gag aag acc      3509
   Asn Gly  Gln Pro His Ser Val  Asn Ile Thr Arg His  Glu Lys Thr
       1110                 1115                 1120
   atc ttt  ctc aag ctc gat cat  tat cct tct gtg agt  tac cat ctg      3554
   Ile Phe  Leu Lys Leu Asp His  Tyr Pro Ser Val Ser  Tyr His Leu
       1125                 1130                 1135
   cca agt  tca tcc gac acc ctc  ttc aat tct ccc aag  tcg ctc ttt      3599
   Pro Ser  Ser Ser Asp Thr Leu  Phe Asn Ser Pro Lys  Ser Leu Phe
       1140                 1145                 1150
   ctg gga  aaa gtt ata gaa aca  ggg aaa att gac caa  gag att cac      3644
   Leu Gly  Lys Val Ile Glu Thr  Gly Lys Ile Asp Gln  Glu Ile His
       1155                 1160                 1165
   aaa tac  aac acc cca gga ttc  act ggt tgc ctc tcc  aga gtc cag      3689
   Lys Tyr  Asn Thr Pro Gly Phe  Thr Gly Cys Leu Ser  Arg Val Gln
       1170                 1175                 1180
   ttc aac  cag atc gcc cct ctc  aag gcc gcc ttg agg  cag aca aac      3734
   Phe Asn  Gln Ile Ala Pro Leu  Lys Ala Ala Leu Arg  Gln Thr Asn
       1185                 1190                 1195
   gcc tcg  gct cac gtc cac atc  cag ggc gag ctg gtg  gag tcc aac      3779
   Ala Ser  Ala His Val His Ile  Gln Gly Glu Leu Val  Glu Ser Asn
       1200                 1205                 1210
   tgc ggg  gcc tcg ccg ctg acc  ctc tcc ccc atg tcg  tcc gcc acc      3824
   Cys Gly  Ala Ser Pro Leu Thr  Leu Ser Pro Met Ser  Ser Ala Thr
       1215                 1220                 1225
   gac ccc  tgg cac ctg gat cac  ctg gat tca gcc agt  gcg gat ttt      3869
   Asp Pro  Trp His Leu Asp His  Leu Asp Ser Ala Ser  Ala Asp Phe
       1230                 1235                 1240
   cca tat  aat cca gga caa ggc  caa gct ata aga aat  gga gtc aac      3914
   Pro Tyr  Asn Pro Gly Gln Gly  Gln Ala Ile Arg Asn  Gly Val Asn
       1245                 1250                 1255
   aga aac  tcg gct atc att gga  ggc gtc att gct gtg  gtg att ttc      3959
   Arg Asn  Ser Ala Ile Ile Gly  Gly Val Ile Ala Val  Val Ile Phe
       1260                 1265                 1270
   acc atc  ctg tgc acc ctg gtc  ttc ctg atc cgg tac  atg ttc cgc      4004
   Thr Ile  Leu Cys Thr Leu Val  Phe Leu Ile Arg Tyr  Met Phe Arg
       1275                 1280                 1285
   cac aag  ggc acc tac cat acc  aac gaa gca aag ggg  gcg gag tcg      4049
   His Lys  Gly Thr Tyr His Thr  Asn Glu Ala Lys Gly  Ala Glu Ser
       1290                 1295                 1300
   gca gag  agc gcg gac gcc gcc  atc atg aac aac gac  ccc aac ttc      4094
   Ala Glu  Ser Ala Asp Ala Ala  Ile Met Asn Asn Asp  Pro Asn Phe
       1305                 1310                 1315
   aca gag  acc att gat gaa agc  aaa aag gaa tgg ctc  att tga          4136
   Thr Glu  Thr Ile Asp Glu Ser  Lys Lys Glu Trp Leu  Ile
       1320                 1325                1330
   ggggtggcta cttggctatg ggatagggag gagggaatta ctagggagga gagaaaggga   4196
   caaaagcacc ctgcttcata ctcttgagca catccttaaa atatcagcac aagttggggg   4256
   aggcaggcaa tggaatataa tggaatattc ttgagactga tcacaaaaaa aaaaaaaacc   4316
   tttttaatat ttctttatag ctgagttttc ccttctgtat caaaacaaaa taatacaaaa   4376
   aatgctttta gagtttaagc aatggttgaa atttgtaggt actatctgtc ttattttgtg   4436
   tgtgtttaga ggtgttctaa agacccgtgg taacagggca agttttctac gtttttaaga   4496
   gcccttagaa cgtgggtatt ttttttcttg agaaaagcta atgcacctac agatggcccc   4556
   caacattctc ttccttttgc ttctagtcaa ccttaatggg ctgttacaga aactagttcg   4616
   tgtttatata ctatttcctt tgatgtccta taagtcggaa aagaaagggg caaagagaac   4676
   ctattatttg ccagttttta agcagagctc aatctatgcc agctctctgg catctggggt   4736
   tcctgactga taccagcagt tgaaggaaga gagtgcatgg cacctggtgt gtaacgacac   4796
   aatcagcaca actggagaga ggcattaaag aaccagggaa ggtagtttga tttttcattg   4856
   aattctacaa gctaatattg ttccacgtat gtagtcttag accaatagct gtaactatca   4916
   gctgcaatac catggtgacc agctgttaca aaagattttt tcctgtttta tctgaaacat   4976
   actggattta tatatgtata agcgcctcaa tggggaatta gagccagatg ttatgatttg   5036
   tttgctcttt ttcttttata gttatagcaa aaatatggat aatttctagt gaatgcataa   5096
   attaggttgc gtttcttatt ttgctttaaa tctctggtag tttttccacc cctgtgacac   5156
   aatcctaata gacagtgtcc tgtaaatgga cacaacacaa taaagtcaag ttattattgc   5216
   tgttactctg gatgatatgg aaaacactgc catattttaa atcaactact ccacgtgttt   5276
   ttccatccaa tcacactgct gtgattcagg gatctttctt ctaaaacgga cacatttgaa   5336
   cctcaggttc atcacaaacc tggtacctgt tgcttcccag aggatggaga agtgtagtta   5396
   atcacacctc ttagtttaat ctgaaatctt gacccagtta tttaacaaat aaatacctca   5456
   ttgattatat ttaaaagtaa tacacttcct gtaaacaaat ggggacaatg catccaaaaa   5516
   atctttttaa acagattaca caaaaattat ttccagaaag gctaccattt atcatcatta   5576
   tatttcaagc ctcttatact taataagcac tttctaaaaa gtcttgagat cccaccattc   5636
   tgaggaattc aatatgatca ctttttcctt ctttgcctgg gagaggttaa gaggcggttt   5696
   cgaaggtata gatgctattg ttctgatggc ccggctgaat aaaatggaaa ttctagtttg   5756
   ttagaattat gcattctttt tcaagattct cagtgtgcct aacttattgg agcacatcag   5816
   tttcttgggt aatggaaaac attacctaga gttgccagtg gcacattaca ccagtacaga   5876
   gcacattcca aaggagacat tggaccagtt aattcccata caagtcaagg taacagaaca 5936
   aaagggaatc ctgatgccct tttaccattg ctggttgagc tcaggcactg tcatggacac 5996
   ccttaatttt aaaaggtttt aatcattctt ctataaaata catttaaaat ggaaaaatac 6056
   ttaatatcac taaatatcag aacaatgtaa catttacaaa tgacatattg aaagcaaagg 6116
   ctgttttatt tagccaagat gattaccatt aggagttact ttatgtattg ttgaaagcaa 6176
   attttaaaca tgatgtttta gaagtgtttc tgatttttaa acctggttta caggtattac 6236
   ttctgcactt accaaataat gccagatgga aatttattat ttcttgcaat tcccgtgata 6296
   gctctgttct ttatgcattg tctcaacact ttcccttttt tcccaaaatg agtagagaat 6356
   taaagccacc caaaacagct tctgctacta aaatgttctc atcctttctc ctccctctcc 6416
   ttttcctgcc acaaaaggtg aaaaatgaga tccaatcctc tcaccaaaat ttcaaaccta 6476
   ggacactgga atgactgcag ggatcagtgg ttctcccata tcaccatcaa ttaagacata 6536
   taggacactg tcttccttca agagggttac aatgtggcca tcagacagga aaccaaacgg 6596
   tggataaagt attaagtaac taagtgccaa ataaatgctg gaaatcttga cctctccttg 6656
   ggattatggg tgtaacaaaa atccctacat ctgtttatga aggccatatt cagtacattt 6716
   taaatggtaa ataatctgtt tatgtgaaga aaaagaatta agtctttctt ccaactctct 6776
   ccttggatag cctagcacag tgcagcctcc ataaccatga cattcccgcc caagctctca 6836
   gtgcctaatc ctgctttgtc attcacatct cacaaaatct tgacatctta cattccaata 6896
   cattatcaag caagcacaag tatgctggta gtagcctctt taaataatat gtatagacaa 6956
   caacaacgac aaaaaataga ctgttttaaa gtttcaggga aagttggtgg ctgatttaaa 7016
   gttgtgcagg aaacatcttc tgtgtatgaa gcaaatgtcg atgttttgaa aagctaggag 7076
   atgactttga atgaatgcaa ggttagtgag atcctaagct ctcaaaatag catattccct 7136
   agagctcaag aaagctggtc caggaggttg aaaaagctat tttgttgtta aattattttc 7196
   tggcccttct taatatttaa aaatgtattt ccccttgtgg ctttcaacca cctgctcaaa 7256
   aaaagagact tgttacatga aagttttcat taaagagctg aaaacaagaa tttagagagc 7316
   cattcctaga aaatgtccta ctgccctgca tttgacaaac aagcatcctt tactaacaag 7376
   agcaggaatt cagaggcaca agaaaaagca ttggcatgag ccaaagagtc tgtcttaatg 7436
   ttacttttga aaatctgctg agcggccacc atatgcaggc tgagagctgg gcacaggcga 7496
   agccattgga agcacttcag gaacaagcac acagctgtgg gacttgaaca tgcaagtgtt 7556
   caggttgtgt caagaagctt ttctttcctt ctatgatgga atctgttctt ttctatccta 7616
   cttttttctc tcttcctctc ctcaccacat tataccctgc tcttacgcag taaacgtttt 7676
   aatggcccgt ttatgtctca tgcctccaaa caacactgaa tttgaaaccc cccatttttt 7736
   cttttcacca ccctgttgag caattttccc aaaaaaaggg cagcaattat taaattgaat 7796
   tcaagtttct agattttact aagttttatt ttgtcaggtt ttttaaattt tttcagtgag 7856
   cgtggtgact gcagaggtta gtgctgtgaa aagctgggct aaatattctt tctgtaaagt 7916
   caaacaggat tccatcccct gtgaaataac acaaaatttc actctctaaa agcaacagca 7976
   tgtaaactag aatgaaagaa ggaaattatg tacgtatgcc taatattctt tgtgaatgtc 8036
   tttcatttaa ctaaaattat attagaaacc agattgataa ataaaaaatt caaagtagtt 8096
   ttaattatcc t 8107
<210> 2
   <211> 1331
   <212> PRT
   <213> Homo sapiens
<400> 2
   Met Gln Ala Ala Pro Arg Ala Gly Cys Gly Ala Ala Leu Leu Leu Trp
   1               5                   10                  15
   Ile Val Ser Ser Cys Leu Cys Arg Ala Trp Thr Ala Pro Ser Thr Ser
               20                  25                  30
   Gln Lys Cys Asp Glu Pro Leu Val Ser Gly Leu Pro His Val Ala Phe
           35                  40                  45
   Ser Ser Ser Ser Ser Ile Ser Gly Ser Tyr Ser Pro Gly Tyr Ala Lys
       50                  55                  60
   Ile Asn Lys Arg Gly Gly Ala Gly Gly Trp Ser Pro Ser Asp Ser Asp
   65                  70                  75                  80
   His Tyr Gln Trp Leu Gln Val Asp Phe Gly Asn Arg Lys Gln Ile Ser
                   85                  90                  95
   Ala Ile Ala Thr Gln Gly Arg Tyr Ser Ser Ser Asp Trp Val Thr Gln
               100                 105                 110
   Tyr Arg Met Leu Tyr Ser Asp Thr Gly Arg Asn Trp Lys Pro Tyr His
           115                 120                 125
   Gln Asp Gly Asn Ile Trp Ala Phe Pro Gly Asn Ile Asn Ser Asp Gly
       130                 135                 140
   Val Val Arg His Glu Leu Gln His Pro Ile Ile Ala Arg Tyr Val Arg
   145                 150                 155                 160
   Ile Val Pro Leu Asp Trp Asn Gly Glu Gly Arg Ile Gly Leu Arg Ile
                   165                 170                 175
   Glu Val Tyr Gly Cys Ser Tyr Trp Ala Asp Val Ile Asn Phe Asp Gly
               180                 185                 190
   His Val Val Leu Pro Tyr Arg Phe Arg Asn Lys Lys Met Lys Thr Leu
           195                 200                 205
   Lys Asp Val Ile Ala Leu Asn Phe Lys Thr Ser Glu Ser Glu Gly Val
       210                 215                 220
   Ile Leu His Gly Glu Gly Gln Gln Gly Asp Tyr Ile Thr Leu Glu Leu
   225                 230                 235                 240
   Lys Lys Ala Lys Leu Val Leu Ser Leu Asn Leu Gly Ser Asn Gln Leu
                   245                 250                 255
   Gly Pro Ile Tyr Gly His Thr Ser Val Met Thr Gly Ser Leu Leu Asp
               260                 265                 270
   Asp His His Trp His Ser Val Val Ile Glu Arg Gln Gly Arg Ser Ile
           275                 280                 285
   Asn Leu Thr Leu Asp Arg Ser Met Gln His Phe Arg Thr Asn Gly Glu
       290                 295                 300
   Phe Asp Tyr Leu Asp Leu Asp Tyr Glu Ile Thr Phe Gly Gly Ile Pro
   305                 310                 315                 320
   Phe Ser Gly Lys Pro Ser Ser Ser Ser Arg Lys Asn Phe Lys Gly Cys
                   325                 330                 335
   Met Glu Ser Ile Asn Tyr Asn Gly Val Asn Ile Thr Asp Leu Ala Arg
               340                 345                 350
   Arg Lys Lys Leu Glu Pro Ser Asn Val Gly Asn Leu Ser Phe Ser Cys
           355                 360                 365
   Val Glu Pro Tyr Thr Val Pro Val Phe Phe Asn Ala Thr Ser Tyr Leu
       370                 375                 380
   Glu Val Pro Gly Arg Leu Asn Gln Asp Leu Phe Ser Val Ser Phe Gln
   385                 390                 395                 400
   Phe Arg Thr Trp Asn Pro Asn Gly Leu Leu Val Phe Ser His Phe Ala
   405 410 415
   Asp Asn Leu Gly Asn Val Glu Ile Asp Leu Thr Glu Ser Lys Val Gly
   420 425 430
   Val His Ile Asn Ile Thr Gln Thr Lys Met Ser Gln Ile Asp Ile Ser
   435 440 445
   Ser Gly Ser Gly Leu Asn Asp Gly Gln Trp His Glu Val Arg Phe Leu
   450 455 460
   Ala Lys Glu Asn Phe Ala Ile Leu Thr Ile Asp Gly Asp Glu Ala Ser
   465 470 475 480
   Ala Val Arg Thr Asn Ser Pro Leu Gln Val Lys Thr Gly Glu Lys Tyr
   485 490 495
   Phe Phe Gly Gly Phe Leu Asn Gln Met Asn Asn Ser Ser His Ser Val
   500 505 510
   Leu Gln Pro Ser Phe Gln Gly Cys Met Gln Leu Ile Gln Val Asp Asp
   515 520 525
   Gln Leu Val Asn Leu Tyr Glu Val Ala Gln Arg Lys Pro Gly Ser Phe
   530 535 540
   Ala Asn Val Ser Ile Asp Met Cys Ala Ile Ile Asp Arg Cys Val Pro
   545 550 555 560
   Asn His Cys Glu His Gly Gly Lys Cys Ser Gln Thr Trp Asp Ser Phe
   565 570 575
   Lys Cys Thr Cys Asp Glu Thr Gly Tyr Ser Gly Ala Thr Cys His Asn
   580 585 590
   Ser Ile Tyr Glu Pro Ser Cys Glu Ala Tyr Lys His Leu Gly Gln Thr
   595 600 605
   Ser Asn Tyr Tyr Trp Ile Asp Pro Asp Gly Ser Gly Pro Leu Gly Pro
   610 615 620
   Leu Lys Val Tyr Cys Asn Met Thr Glu Asp Lys Val Trp Thr Ile Val
   625 630 635 640
   Ser His Asp Leu Gln Met Gln Thr Pro Val Val Gly Tyr Asn Pro Glu
   645 650 655
   Lys Tyr Ser Val Thr Gln Leu Val Tyr Ser Ala Ser Met Asp Gln Ile
   660 665 670
   Ser Ala Ile Thr Asp Ser Ala Glu Tyr Cys Glu Gln Tyr Val Ser Tyr
   675 680 685
   Phe Cys Lys Met Ser Arg Leu Leu Asn Thr Pro Asp Gly Ser Pro Tyr
   690 695 700
   Thr Trp Trp Val Gly Lys Ala Asn Glu Lys His Tyr Tyr Trp Gly Gly
   705 710 715 720
   Ser Gly Pro Gly Ile Gln Lys Cys Ala Cys Gly Ile Glu Arg Asn Cys
   725 730 735
   Thr Asp Pro Lys Tyr Tyr Cys Asn Cys Asp Ala Asp Tyr Lys Gln Trp
   740 745 750
   Arg Lys Asp Ala Gly Phe Leu Ser Tyr Lys Asp His Leu Pro Val Ser
   755 760 765
   Gln Val Val Val Gly Asp Thr Asp Arg Gln Gly Ser Glu Ala Lys Leu
   770 775 780
   Ser Val Gly Pro Leu Arg Cys Gln Gly Asp Arg Asn Tyr Trp Asn Ala
   785 790 795 800
   Ala Ser Phe Pro Asn Pro Ser Ser Tyr Leu His Phe Ser Thr Phe Gln
   805 810 815
   Gly Glu Thr Ser Ala Asp Ile Ser Phe Tyr Phe Lys Thr Leu Thr Pro
   820 825 830
   Trp Gly Val Phe Leu Glu Asn Met Gly Lys Glu Asp Phe Ile Lys Leu
   835 840 845
   Glu Leu Lys Ser Ala Thr Glu Val Ser Phe Ser Phe Asp Val Gly Asn
   850 855 860
   Gly Pro Val Glu Ile Val Val Arg Ser Pro Thr Pro Leu Asn Asp Asp
   865 870 875 880
   Gln Trp His Arg Val Thr Ala Glu Arg Asn Val Lys Gln Ala Ser Leu
   885 890 895
   Gln Val Asp Arg Leu Pro Gln Gln Ile Arg Lys Ala Pro Thr Glu Gly
   900 905 910
   His Thr Arg Leu Glu Leu Tyr Ser Gln Leu Phe Val Gly Gly Ala Gly
   915 920 925
   Gly Gln Gln Gly Phe Leu Gly Cys Ile Arg Ser Leu Arg Met Asn Gly
   930 935 940
   Val Thr Leu Asp Leu Glu Glu Arg Ala Lys Val Thr Ser Gly Phe Ile
   945 950 955 960
   Ser Gly Cys Ser Gly His Cys Thr Ser Tyr Gly Thr Asn Cys Glu Asn
   965 970 975
   Gly Gly Lys Cys Leu Glu Arg Tyr His Gly Tyr Ser Cys Asp Cys Ser
   980 985 990
   Asn Thr Ala Tyr Asp Gly Thr Phe Cys Asn Lys Asp Val Gly Ala Phe
   995 1000 1005
   Phe Glu Glu Gly Met Trp Leu Arg Tyr Asn Phe Gln Ala Pro Ala
   1010 1015 1020
   Thr Asn Ala Arg Asp Ser Ser Ser Arg Val Asp Asn Ala Pro Asp
   1025 1030 1035
   Gln Gln Asn Ser His Pro Asp Leu Ala Gln Glu Glu Ile Arg Phe
   1040 1045 1050
   Ser Phe Ser Thr Thr Lys Ala Pro Cys Ile Leu Leu Tyr Ile Ser
   1055 1060 1065
   Ser Phe Thr Thr Asp Phe Leu Ala Val Leu Val Lys Pro Thr Gly
   1070 1075 1080
   Ser Leu Gln Ile Arg Tyr Asn Leu Gly Gly Thr Arg Glu Pro Tyr
   1085 1090 1095
   Asn Ile Asp Val Asp His Arg Asn Met Ala Asn Gly Gln Pro His
   1100 1105 1110
   Ser Val Asn Ile Thr Arg His Glu Lys Thr Ile Phe Leu Lys Leu
   1115 1120 1125
   Asp His  Tyr Pro Ser Val Ser  Tyr His Leu Pro Ser  Ser Ser Asp
       1130                 1135                 1140
   Thr Leu  Phe Asn Ser Pro Lys  Ser Leu Phe Leu Gly  Lys Val Ile
       1145                 1150                 1155
   Glu Thr  Gly Lys Ile Asp Gln  Glu Ile His Lys Tyr  Asn Thr Pro
       1160                 1165                 1170
   Gly Phe  Thr Gly Cys Leu Ser  Arg Val Gln Phe Asn  Gln Ile Ala
       1175                 1180                 1185
   Pro Leu  Lys Ala Ala Leu Arg  Gln Thr Asn Ala Ser  Ala His Val
       1190                 1195                 1200
   His Ile  Gln Gly Glu Leu Val  Glu Ser Asn Cys Gly  Ala Ser Pro
       1205                 1210                 1215
   Leu Thr  Leu Ser Pro Met Ser  Ser Ala Thr Asp Pro  Trp His Leu
       1220                 1225                 1230
   Asp His  Leu Asp Ser Ala Ser  Ala Asp Phe Pro Tyr  Asn Pro Gly
       1235                 1240                 1245
   Gln Gly  Gln Ala Ile Arg Asn  Gly Val Asn Arg Asn  Ser Ala Ile
       1250                 1255                 1260
   Ile Gly  Gly Val Ile Ala Val  Val Ile Phe Thr Ile  Leu Cys Thr
       1265                 1270                 1275
   Leu Val  Phe Leu Ile Arg Tyr  Met Phe Arg His Lys  Gly Thr Tyr
       1280                 1285                 1290
   His Thr  Asn Glu Ala Lys Gly  Ala Glu Ser Ala Glu  Ser Ala Asp
       1295                 1300                 1305
   Ala Ala  Ile Met Asn Asn Asp  Pro Asn Phe Thr Glu  Thr Ile Asp
       1310                 1315                 1320
   Glu Ser  Lys Lys Glu Trp Leu  Ile
       1325                 1330
<210> 3
   <211> 401
   <212> DNA
   <213> Homo sapiens
<220>
   <221> allele
   <222> (201)..(201)
   <223> A/G
<400> 3
   tgaagactag agtcagggac ggtttcagag ggaagatggg tcattagctg gatctcaggt     60
   atttgagatg atcacatgta tttctcaacc cgtctcctct ggagaagtgg aatttttggt    120
   ccatttcatt tctggtatgt ctatttcttt attaaccatt ttcaaatttc ctcttttgtc    180
   tgtgactttc aacagcccaa rtctgtctct atctcaagtc ctcccacgcc acccccctcc    240
   aagtccctgt ctgtgttcca atcccctgcc tctcctaacc tctcttcaca ctcttctctt    300
   ccaaagactc cccccaggta cagtgccttc gagcctacaa gccccgagag aatgatgaat    360
   tggcactgga gaaagccgac gtggtgatgg tgactcagca g                        401
<210> 4
   <211> 604
   <212> DNA
   <213> Homo sapiens
<220>
   <221> allele
   <222> (478)..(478)
   <223> C/T
<400> 4
   tcctataagc atcccttggt aaccaatatt attttcttta ccaaaattca gagagaaaaa      60
   tgaacaaatg ttatggagcc caaactatca gagtcagaag aggccccttt tccttcaaac     120
   aacacctatg aagttataca cactgctttg ggggaaatca atcaattaca atgctcactt     180
   cccactagag ggagcctcct atctttctga ggaaatcttg aaaaggcaat ttaaaaattc     240
   aggaacagta ctgcaagata atctattgtc agtattacca gttcacacag cttatttatt     300
   aaaactcaaa ttatttttca tagttcttta tgtttttact agtgcaaaca caaaagtaaa     360
   actacacagt aatacagagt gaatctcatg tgaatattgc cctgatacga acttcaatat     420
   aggaagctct taactcttga gtcatttggg acttgggact tctgtgagat gatgtttytg     480
   aacactggtg atatacacaa aaatatgtat cgatctagac actgttacat agttggtaga     540
   tcagggtggt gatctttgag ggattcacag caaagtcaat tgaaatctct acctctcctg     600
   aata                                                                  604
<210> 5
   <211> 436
   <212> DNA
   <213> Homo sapiens
<220>
   <221> allele
   <222> (236)..(236)
   <223> A/G
<400> 5
   tgctgtcagc accatcagga gaatttatca ccacacaaat aataataata ataataataa      60
   taataataat aaatgtgtca ctgcaattcc tactagaaac tcaactgcca tcagagtgca     120
   caaagtccca aatttatata aggaacagct gggtcacttc catgacattc aagcttgggc     180
   tggagtagcg acgttttgtc taagccaact cagaaccctt tataatactc ctcccrccta     240
   taattttctg ttttaggctc tctgaaatat cctccctttc tgttaccttt acctgaccat     300
   tacctcgagg aatctctcaa ggctcacact cggaatcttt tttactgcag cttgtagtcc     360
   acagtagctg gctctttact ttgggcacag ctattgatct ctgcccggaa gtatggactt     420
   gatcttggga cttccc                                                     436
<210> 6
   <211> 628
   <212> DNA
   <213> Homo sapiens
<220>
   <221> allele
   <222> (247)..(247)
   <223> C/T
<400> 6
   agcttcactc aaacataaat tatgatttag tatgtcttga aggtatcatt ttgcctgaca      60
   gtataagcaa aaagcagaaa atagtgattg attaacctta ttttgtagtg atgcaggaat     120
   ctaataaacc caaattaagg attgaaatga agataacaaa attgaagtaa ccaatatttc     180
   tggttggagc ccacttttat tacagcgcta aaaatagtca ttgacataag aaaggataga     240
   tgaatgycat cttgttattt tccagcactg agataggttt agatgagtat gtcggagata     300
   ggaacattaa gaagttggta gccctcgcac atatgatgaa taaagataga tcattataaa     360
   tggcttcaaa actcttttct tcgaaggttc ttactgggct tattataaaa ggaacaaaga     420
   ttgttagaag atatgctgtc ttcactgtct caaaaacaat actttttgaa accttctcaa     480
   gaatatagtg aaatggataa agaacctctc tgagaaactc ctgttgtcat tggattttga     540
   aagttgtcat aaaccacctc tgtacctaac ctaaataatg aatgttgttg tctttgttgt     600
   atttaaaata taagtattgc taccaagt                                        628
<210> 7
   <211> 466
   <212> DNA
   <213> Homo sapiens
<220>
   <221> allele
   <222> (201)..(201)
   <223> A/G
<400> 7
   gtaactataa ttgtactatt ttcctgtttt tttttctttt gtcttctttg gctattttga      60
   tgccttttaa tgcatccccc tgttgatgta cagtttaata tctgatataa cactaagatt     120
   aattttttaa ttgttaatat atctttgact agtctttccc tatattatta ccattgtgat     180
   tacttttact tactacgatc raagtagcaa cttgtagtca gatagcagtg atattcacgg     240
   tctatcaatt tatccattca tccatttatc catcctactt ttcttccctt cccccatcca     300
   tccattatgt actgtggatt tgcactgaag tcaattggaa ttaagtatta taaaactcta     360
   ttttactaat agccatataa aacccttaag aggcaattca gacattcaca atacttggaa     420
   ttttaacagt tacctctatt gactattaca atatagcata atctgt                    466
<210> 8
   <211> 401
   <212> DNA
   <213> Homo sapiens
<220>
   <221> allele
   <222> (201)..(201)
   <223> C/T
<400> 8
   aacatcttga atgtagtgtt agttatgatg atatataccg tttctaaaat ttctccatat      60
   gcacattgaa aatgaataca ttttattaca tgtaaattct taagagatgt aattttaaaa     120
   aagagctact gaaatgctaa acaagacaga aaaatgtccc tcgagattct tgctattgag     180
   ctgacgacca aattcaggca yttgcagagt agttgacctt ctcttaagaa ggagccctcc     240
   tctgtgccct cactataaga aggagcctcc caacattaga ttttctcttg aagagccctt     300
   tggactgttg ttctgtctga gagctactgg agaatctctt tgatagcttt gcttttgacg     360
   gttttctctg gagaaaggtc taatagaagc aaacagttaa a                         401
<210> 9
   <211> 717
   <212> DNA
   <213> Homo sapiens
<220>
   <221> allele
   <222> (466)..(466)
   <223> A/G
<400> 9
   tcaggttggg agagaaatga tattgaaggg acaaaatagc aaacataaga agccaccttt     60
   gctcatttct gcttcccagc ataatttccc aaagcgcttg gccctgtgac aacatacagt    120
   gctccaaaaa aaaaaaaaaa aaaagaggtc gaaacaaaat aaaacatacc ccactacgtc    180
   tcttgcatga gtcagtattc cttaaaaaat aaatgaccct aatcattgct tttttctaac    240
   ataaaataat gtctgatagg attaaaaatt atgcctctat aatctgtaat catgaatcat    300
   ataatctatc agtatcatat aatctataag atcatatctt atcatataat ctatatgagt    360
   gtagcttata gattacatct tatagataag atatactctt gtccctcatt accttagggg    420
   aagctctcgt tcactggcag attgccatgg ctaattccag ctgccrttca ttcaaatgtt    480
   aatacggata taggatttta tttatttatg gtagagttgt aggaaaacaa aatgtctcta    540
   ttaacatttc tctattttgc taaaatattc tgaaattata ggctctgata aataatatgt    600
   tatatcttat aggtaagata cactgttaca tccaaacttt aatataattt tacaggtact    660
   aaccccccat tacctatatg taaacatcag aatcaaaaat tgatcccttg acttaac        717
<210> 10
   <211> 401
   <212> DNA
   <213> Homo sapiens
<220>
   <221> allele
   <222> (201)..(201)
   <223> C/T
<400> 10
   caatatatga ggagataaaa tgaattttcc acaaactatg attctgtgtc atctaaacat     60
   tagcttgcac aggcctttta aagaaagcag agtgaggact ccctggccag tgttctctac    120
   catctcttct gcctaccttc ttttctctca tggaagtaag aaaagaatcc atttcatcaa    180
   aggttgaaca ttccacttca yccctgaatt ctctcttgct ttgagttctt aggtacatct    240
   atattagata tcactttctc ctctgcatcc ccaatgccct tttccctcct cagcatacct    300
   gatcctctgt ccttgctgac ctttgtatgt gtgtgttttc tcccttgatg acatatccct    360
   cttcagctat tgctctattt atattcagaa tcccaggcaa g                         401
<210> 11
   <211> 692
   <212> DNA
   <213> Homo sapiens
<220>
   <221> allele
   <222> (211)..(211)
   <223> A/G
<400> 11
   agatagatga tagatagata gatagataga tagatagata gatagataga ggagagagag 60
   agagagagag agattagatg gataggtatg tagtttcacc tataagcaaa ggcaaaaatg 120
   agtatctcag aaaggcagat ttagggacca ctaagatttt aagttgcatc attctgtaaa 180
   ttggagaata ttatcactta ctgtactgtc rcaaatttca gataagttaa cattcctgaa 240
   tgttcttggc acagcataaa tgctcaatat cttcaattat catacttttt ccccttcatc 300
   cctgccctga cgcatgatag atcatttctt aaaatatact acagcatgta ttaaataata 360
   tttctactgc tcagttttct ggggaaaata gtgtttagtt aatttctagg ttttgttaag 420
   aatttacaaa atatatttca taaatgtgtt cttttccttt cagaaataaa tgtataattt 480
   tactcacaac atttcaacaa ctttcctaac ttgcatactt catttttcta gtgtttcata 540
   aaagttctaa atttaatcat atgatatttt aaaataagat attaagcaag aaaacacagc 600
   atctattgta tttttaaacg aatcttatgg tttttatttt attatttcaa ttactgttac 660
   taaattatta attctaggtt agaattataa ag 692
<210> 12
   <211> 401
   <212> DNA
   <213> Homo sapiens
<220>
   <221> allele
   <222> (201)..(201)
   <223> A/G
<400> 12
   tgtttgaaat attagaagta tttcataagg ctcctatatc tgtttttcta gactacatat 60
   tgagaactgc tgtaattaca gtatcatcct cagtgttaga tgtaaaatgt taagaaaaat 120
   ctgtccatct ggcaagagga ggtaaaagtt tgttagagtg ggcagaagtt aggtctgata 180
   gcacctaggt ctagagaaac rcaaactcca ggtaatacat atattctagt aaggtttgct 240
   ccatttaaaa aatacaaact tatagaaaca gaaaggcatt tattatgctc atttagtagc 300
   aataaatgcc ttctaatatc tttgaaaatt tatcatgcat tctcttctta aatgtcttca 360
   atgtgtacag agcccatagt taataacaat tctaattatt a 401
<210> 13
   <211> 401
   <212> DNA
   <213> Homo sapiens
<220>
   <221> allele
   <222> (201)..(201)
   <223> A/C
<400> 13
   ctgttttaaa ttcataaaac tgttataaat ttatgtgata tagctaatat gaaagacacc     60
   tgaattaaac actttaatga aatgaatatt tattatcaga cagaggttta gaataagaaa    120
   tgtcaatata tagaactgcc tccactaaac ctttttcctg atgaatttaa gagatgtgtt    180
   tattgaacag aagcacaatt matagataac tcatctttat agggcgtctg ttgccatggt    240
   aatagtgatg tgacatcgtt caaagggaag tgtttgttct aaaagcaatg atgagtaaca    300
   tcctgcagcc tccattcaaa actaaaggta ggtaggttgg gctgttttcc taggcacaga    360
   caagggtgta gaatcctgac aatctctctg aagtacaccc c                        401
<210> 14
   <211> 628
   <212> DNA
   <213> Homo sapiens
<220>
   <221> allele
   <222> (428)..(428)
   <223> A/G
<400> 14
   tagtttacaa aatatgttta catatatcat tgaatttgat actcctaaga accccataag     60
   caaagtatga tctcttatta tacatattct atagataaga aactgagcat ctagaagtta    120
   agtgactgct caagatcacg tggctaggag tttacccaaa gcctaaactt aggtcttctg    180
   atttctaatc tagtgtttct cctactataa tatttcagtc tgctttttcc tttcactcta    240
   aggcttatta ttttcactaa attcaaattg ccttttctgc tttgttaaat actagagtta    300
   gacatttgta aagttaaaca cacacacaca tacacacaca cacacacgac attaagggca    360
   agatatatac ttccaaggtt tctgagaggt acagcttata caaggaaaat cttttgtaag    420
   aaattgtrta gatctactta ttcatgtgct gagtacacat tttcaatttt gactattggt    480
   ataatttaga gaaattcaat taaaaagaaa aaaataagta ctcctcttta ggcatttatt    540
   ctgttcacct gaaagtatat tcatagccat tttttttttg ttaatgttaa ataacagatc    600
   cctttccagg gatcctttcc agggatcc 628
<210> 15
   <211> 663
   <212> DNA
   <213> Homo sapiens
<220>
   <221> misc_feature
   <222> (137)..(137)
   <223> N = A, T, C or G
<220>
   <221> allele.
   <222> (113)..(113)
   <223> A/G
<400> 15
   gctaaatgtt gcataataca tgtgggatat aagggatcca actttaagga cttaaaaaag 60
   gctaaaggtg ttatatatct aaaaaacatt ctctgtattt tatgaactgg agrtaagagt 120
   catagcagaa acttccnacc attagtctcc ttggcaatac tgattaacct ccctcagtca 180
   caattttgca caaaaagaat agaaatgtta aagaaatgct ttgggatttc aaggctatca 240
   ttaacattta tgggaagaga ggtgaaagag ctagctagta attatcaatg tactgcattg 300
   ctctacatct ctggcaggaa cccaactttt tggatcaact agcaggcagc accgttagcc 360
   atgagcttcc atggtcacga tgagattgta tgtcttggca tgattcggca ggtgtcattt 420
   atatgcaatg tcatctgcag gaaaaatgca gggagtatca ttctccctgc ttaagttctt 480
   ttatggagac aactcaaagt cttcgctaaa tgattactct tgatcatttt cagttacaac 540
   aatgacttaa gagtattgtg gaaaagcatc ccagtgaaca gaactgttaa gatgaataaa 600
   aatgtctaaa ttctaaatat atattttcaa ctatttgcaa ggctatatga acatttttga 660
   aat 663
<210> 16
   <211> 3389
   <212> DNA
   <213> Homo sapiens
<220>
   <221> allele
   <222> (3189)..(3189)
   <223> A/C
<400> 16
   ttggtgaagc tatacttact gttttttaaa gaaaaaagct tatcataact tatttaaaag 60
   acaaattttg ttttgttttg ttttggtttt ttattttttt attttttatt tttatttttt 120
   aattttatta ttatacttta agttttaggg tacatgtgca caatgtgcag gttagttaca 180
   tatgtataca tgtgccatgc tggtgcactg cacccattaa ctcatcattt agcattaggt 240
   atatctccta aagctatccc tcccccctcc ccccacccca caacagtcca cagagtgtga 300
   tgttcccctt cctgtgtcca tgtgttctca ttcttcaatt cccacctatg agtgagaata 360
   tgcagtgttt ggttttttgt tcttgcgata gtttactgag aatgatgatt tccaatttca 420
   tccatgtccc tacaaaggac atgaactcat catttcttat ggctgcatgg tattccatgg 480
   tgtatacgtg ccacattttc ttcatccagt ctatcattgt tggacatttg ggttggttcc 540
   aagtctttgc tattgtgaat agtgccgcaa taaacatacg tgtgcatgtg tctttataac 600
   agcatgattt atagtccttt gggtatatac ccagtaatgg gatggctggg tcaaatggta 660
   tttctagttc tagatctctg aggaattgcc acactgactt ccacaatggt tgaactagtt 720
   tgcagtccca ccaacagtgt aaaagtgttc ctatttctcc acatcctctc cagcacctgt 780
   tgtttcctga ctttttaatg attgccattc taactggtgt gagatggtat ctcgttgtgg 840
   ttttgatttg catttctctg atggccagtg atgaagagca ttgtttcatg tgttttttgg 900
   ctgcataaat gtcttctttt gagaagtgtc tgttcatgtc ctttgcccac tttttgatgg 960
   ggttttttgt ttttttcttg taaatttgtt tgagttcatt gtagattctg gatattagcc 1020
   ctttgtcaga tgagtaggtt gggaaaattt tctcccattt tgtaggttgc ctgttcactc 1080
   tgatggtagt ttcttttgct gtgcagaagc tctttagttt aattagatcc catttgtcaa 1140
   ttttggcttt tgttgccatt gcttttggtg ttttagacat gaagtccttg cccatgccta 1200
   tgtcctgaat ggtaatgcct aggttttctt ctagggtttt tatggtttta ggtctaacgt 1260
   ttaagtcttg aatccatctt gaattaattt ttgtataagg tgtaaggaag ggatccagtt 1320
   tcagctttct acatatggct agccagtttt cccagcacca tttattaaat agggaatcct 1380
   ttccccattg tttgtttttc tcaggtttgt caaagatcag atagttgtag atatgcagcg 1440
   ttatttctga gggctgtgtt ctgttccatt gatctatatc tctgttttgg taccagtacc 1500
   atgctgtttt ggttactgta gccttgtagt atagtttgaa gtcaggtagc gtgatgcctc 1560
   cagctttgtt cttttggctt agggttgact tggcgatgca ggctcttttt ttggttccat 1620
   atgaacttta aaatagtttt ttccaattct gtgaagaaag tcattggtag cttgatgggg 1680
   atggcattga atctataaat taccttgggc agtatggcca ttttcatgat attgattctt 1740
   cctacccatg agtatggaat gttcttccat ttgtttgtat cctcttttat ttccttgagc 1800
   agtggtttgt agttctcctt gaagaggtcc ttcacgtctc ttgtaagttg gattcctagg 1860
   tattttattc tctttgaagc aattgtgaat gggagttcac tcatgattta gctctctgtt 1920
   ggtctgttat tggtgtataa gaaagcttgt gatttttgta gattgatttt gtatcctgag 1980
   actttgctga agttgcttat cagtttaagg agattttggg ctgaaacaat ggggttttct 2040
   agatatacaa tcatgtcgtc tgcaaacagg gacaattcga cttcctcttt tcctaattga 2100
   atacccttta tttccttctc ctgcctaatt gccctggcca gaacactatg ttgaatagga 2160
   gtggtgagag agggcatccc tgtcttgtgc ccgttttcaa agggaatgct tccagttttt 2220
   gcccattcag tatgatattg gctgtgggtt tgtcatagac agctcttatt attttgagat 2280
   atgtcccatc aatacctaat ttatggagag tttttagcat gaagggttgt tgaattttgt 2340
   caaaggcctg ttctgcatct attgagataa tcatgtggtt tttgtctttg gttctgttta 2400
   tatgctggat tacatttatt gatttgcgta tgttgaacca gccttgcatc ccagggatga 2460
   agccctcttg atcatggtgg ataaggtttt tgatgtgctg ctggattcgg tttgccagta 2520
   ttttattgag gatttttgca tcaatattca tcaaggatat tggtctaaaa ttcgcaaata 2580
   gacgcaataa aaaatgataa aggagatatc accaccgatc ccacagaaat acaaactacc 2640
   atcagagaat actacaaaca cctctacaca aataaactag aaaatctaga agaaatggat 2700
   aaattcctcg acacatacac cctcccaaga ctaaaccagg aagaagttga ctctctgaat 2760
   agaccaagaa caggctctga aattgtggca ataatcagta gcttaccaac caaaacgagt 2820
   ccaggaccag atggattcac agccgaattc taccagaggt acaaggagga actggtacca 2880
   ttccttctga aactattcca gtcagtagaa aaagagggaa tcctccctca gtcattttat 2940
   gaggccagca tcatcctgat accaaagccg ggcagagaca caaccaaaaa agagaatttt 3000
   agagcagaaa tttttttaaa aagaaattca aaaaaatcat ttattaaaat aggcaagtac 3060
   ttctaaaagc agggcttgaa ggatgcactg atatgtactt tgataatgta agccccaaag 3120
   atcaggacag gtgtctgtct tatttgctct tctatctcat ttacaattcc ctgaatgaat 3180
   aatgaatgma tgtgtgtatc acaagaaaaa aatcatggct atggttgcca ttcgaaagag 3240
   gcacagagtt ataacactag tcataggtag tgcagataga agcaagatca ttgcatgggg 3300
   gctggaggga acttaaataa ctagtagatt acatataata acctcgattc tcatccctca 3360
   ttctatgtca ggcttagagg gtctgcata 3389
<210> 17
   <211> 401
   <212> DNA
   <213> Homo sapiens
<220>
   <221> allele
   <222> (201)..(201)
   <223> A/C
<400> 17
   gttaacttat aatctctcac gcaatggttt ctcctagtgt ttaataaaat ggtattaagg 60
   gtaactgcta ctattttttc taaaattaga aggtaacatt gaatggacac acagcagata 120
   catgttacat tctgaaatgt atgctttgct ggagcagaat gaatgcaatc aatgtaatgg 180
   aaatgctgca ctaatggctc mttaaacatg gaaagtatgc atagatgaaa aaagtctgtg 240
   catatgtttc agttcactga aggaagcaat gaactataat gtggaaagaa aaggaaacat 300
   acttctttac gtaaatattc ttacttctgg aaaagaattg ttgaatcagt caggaatttg 360
   acagcaagat ttcaactatt ggagcagaca actttatatt g 401
<210> 18
   <211> 201
   <212> DNA
   <213> Homo sapiens
<220>
   <221> allele
   <222> (100)..(100)
   <223> G/C
<400> 18 <210> 19
   <211> 1679
   <212> DNA
   <213> Homo sapiens
<220>
   <221> allele
   <222> (1373)..(1373)
   <223> C/G
<400> 19 <210> 20
   <211> 606
   <212> DNA
   <213> Homo sapiens
<220>
   <221> allele
   <222> (501)..(501)
   <223> A/G
<400> 20
<210> 21
   <211> 401
   <212> DNA
   <213> Homo sapiens
<220>
   <221> allele
   <222> (201)..(201)
   <223> A/C
<400> 21
   ttacatgttc ctggtttaat cataagacaa atggcctctc tcttctggta acaattttat      60
   attcctggtc aagggagcta ggttggccca cacatgagag ggaacagtgg aagtaggatg     120
   tgtaatgccc agaagaacag gcatcggagg agagcccagg agttgattct atatgtaaat     180
   ctgttataat accaatccga mcaagctagt attctatcaa atagccaaat gcagaggtaa     240
   gcaggaagta agtaatatct tttgtgattt tttaaaatga cacacagtag atgagctcag     300
   aacatataca gtacagtagc cactgggcaa ccattagata aacaagaaaa gagccagacc     360
   atggggatga aaccatcttt aatcatggca agtggaataa t                         401
<210> 22
   <211> 605
   <212> DNA
   <213> Homo sapiens
<220>
   <221> allele
   <222> (307)..(307)
   <223> C/T
<400> 22
   caatatgatg ttctgacagc acagtaggaa gcaatgtcaa gtgaactaaa cacaaccttt      60
   catggaaatt gttgcttaga atgagaataa gattttaaaa agggagtcca ttaaaaacat     120
   atttattaaa taattaaata ggtggtctgg ggcttggtga aacttctgaa gacaatgcac     180
   aaatcactaa agtttgagaa ttgctttctt aaggtaatta actatctatc agaaattggc     240
   tataaatggc atattaataa tgtaaacttt tctcattttt caataattca atgtctctga     300
   cagcaayaac tgtatcactg gcatattata acagattatt tgaaataatt ttatacatac     360
   ttataacatt ataagttcca cacaatcaga ttttatgtgt gtttgtcact gtgtacctat     420
   gcctgaaaca taagatgcac tcataaatat tggttaagta aatacattaa caattatgca     480
   agcttattta aacacattaa aattctaaaa actgtacctg ggagataaca aagaatgata     540
   aacatagata tttcataagt ctgggaaacg attcacaccc tctctctgct atctgataaa     600
   acccc                                                                 605
<210> 23
   <211> 401
   <212> DNA
   <213> Homo sapiens
<220>
   <221> allele
   <222> (201)..(201)
   <223> C/T
<400> 23
   ctgctaaccg atgaccctat ccccaaagct gtttccattc attctctttg ttgagagtat 60
   gtcaaaccat cccaggatgg aggctggaga tgagtttaac cccacctgca actgttctct 120
   cgatggagct actctacaga aaaatcatgt tccattagtt cactaaatca taacaaaagt 180
   aaatgtactt gtttgaacat ygtacaaact agtcctaaaa gggttctcac gtattttata 240
   tgcgcagtct ttagggaggc aaaaatgttt tatgcaatct atacaagaaa gttatatttt 300
   cagctgttat ccaaaacaaa ggaagcgtac tctgtgaaaa attggtatta ttgtttaaca 360
   atatccattt ccagaggaat gcttttctta agtagaagtt g 401
<210> 24
   <211> 844
   <212> DNA
   <213> Homo sapiens
<220>
   <221> allele
   <222> (344)..(344)
   <223> C/T
<400> 24
   tgagtgaaag gcgtcaggaa agccatttgt aaaatgatga tgaggacgtg cttcttgcct 60
   gcaggttaat cccaaggtga tcacaaatag ctgcacgatg atgccaggca caatttgatg 120
   tatgggaggc agcaagcagg gatgaagaaa gcatgaactg tggtcacaaa gatctacttc 180
   tatgtgttgg cttcgccgct catggcatgt gagcttccca ggtcctattt gggcctcagt 240
   ctccttatgt gtggcattgg cataacaata tgcaccgcgt aggagtgtgt taataacaga 300
   taatgcactt ctttgcacta attctagtta ctggtagctg ttaygattat tgctattatt 360
   gagtataggc acattctata gaagcacata gttggaataa attaaatatg gctgagatga 420
   tgtgtatata aatctgatac tagaggtatc aggagtgcta tggactgaac tgtgtcccct 480
   caaaatttgt ctgttgaatc cctaacctcc aatgtgactg catttgaaga tgggaccttt 540
   aaagaagtac ttaacattaa atgaggccat aaaggtgaga cctaatccaa tagaactggt 600
   gttcttgtaa gaagagggac agacactggg gatgctcttg cacagagaaa agatcaagtg 660
   aagacacggc aagaagacag cagtctgaaa accaagaaga gaagcctcag aagaaagcaa 720
   ccctacggac acactgatct tggacttcca gcttttagaa ctgtgagaat gtttattgtt 780
   taagccacca gactatggta ttctgttagg gcagcccaag cagaccaaga caatgaggaa 840
   gggt 844
<210> 25
   <211> 701
   <212> DNA
   <213> Homo sapiens
<220>
   <221> allele
   <222> (501)..(501)
   <223> C/T
<400> 25
   tacaggtttc gggtttcaca gaaacaacag caaacccaat agtgcttcac tcagtttccc 60
   tcccaaagac agcaaagaga gaaaattaac tacctggcca ggcgcagtgg ctcacgcctg 120
   taatcccagc actttgggag gccgaggctg gaggatcacc tgaggtcagg agtttgagac 180
   cagcctggcc aacatggtga aaccccgtct ctactaaaaa tacaaaaatt agccaggcat 240
   ggtggtgggt gcctgtaatc ccagctactt gggaggctga ggcaggagaa tcgcctgaac 300
   ccgggaagtg gagattacag tgagctgaga ttgcaccact acacttcagc ctgggtgaca 360
   gagtgagact ccgtctcaaa aaaaaaaaaa agagaaaaca aaaaaccaac tatctaaggc 420
   agaatggtaa taaactgccc agtaatgctt tcagggataa ttaaatgagt gatgagggca 480
   caattacaaa taagctaacc ygagttactt ggaaagcaga tggaagcacc caagaccata 540
   caacaagaat gaaaccccag aacagtaaac acacggtatt cctaccatat atgcccccca 600
   tcggtgtcac ctaaaccaac atctctttga cctttttttt actcatgtga ccagttattt 660
   gattgtttgg ctgtcaaagt cacaccaaga taataaatta g 701
<210> 26
   <211> 953
   <212> DNA
   <213> Homo sapiens
<220>
   <221> allele
   <222> (453)..(453)
   <223> A/G
<400> 26
   acatgtaccg gatcaggaag accagggtgc acaggatggt gaaaatcacc acagcaatga      60
   cgcctgacgg agaaaggaga agcacgagag gttagacagg gcttgggagt ccctccaaca     120
   ctgtccccac ttcactacag ctccgtcaga cacagccctc tcccatataa aacacaacct     180
   ccaaaacaac aaagataaca aaaatgaagt aagatgtctg tttttgtcca tgaaggagta     240
   acaggaaaaa caggagtaac ttgtcttcca gccaaaagta actaaaatac tggacacagt     300
   ctatgaaatg acagtctaca gacattagaa aacaggcaga aaggaaccat gatgtctgag     360
   aacagagaaa caaaaaggag gaatcctaca tttttcctag ttttctggct ggcagcgatt     420
   tttggacagc agtgtaggga ggggcagcac aaraaagcac agtggtttcc ctgaatggag     480
   aggggagata gagtgggcgc gcgagagagc tatgtataga aagaacccta ataatctgca     540
   cataagtctc tctgaatttg tggccgaata atactaacat gtgcatgtac agaataaaag     600
   tccacaggcc aggcaaaaaa ccagagctca cggagggctg ggagatgttt atagtccaac     660
   aaccacactg aagagacctc attgaacaat cagagcattt aatacagttg atagaatggt     720
   catgctgaag gtgtttgcta aaataggcct agaataaaga ctactctagc tctgtcctga     780
   aaaagcttta aaacacatct aaataacgtc aagctgcaga gaaaaataat tcagagaaaa     840
   aaaatgtagc aaaatgtcac ttgaccagaa gaaacatcag tcaattagaa ggagagccag     900
   aaaggacaga gcatggaatg agcagatgag gacagtacaa tagctattag aaa            953
<210> 27
   <211> 718
   <212> DNA
   <213> Homo sapiens
<220>
   <221> allele
   <222> (518)..(518)
   <223> C/G
<400> 27
   gatttaataa atctgccact gaatcaaaat acaggagcct tgtcaccttg aagttgcata     60
   catgtgttgt ccaatacggt ggccgccagc cacacatggc tattgaccac ttgagatgtg    120
   gcttgtctga actgagatat gctttaaaca taaaatacac actgcatttt aaagacttag    180
   catgaaaacg aagaatgtca aatagctcat taagtaattt ttacatgttg tttacatgtt    240
   acaaagatat tttcaacata ctgagaaatg aaacgttgtt aaaattgatc ttacctgtct    300
   gtttttactt tttaatcttg ctactagaaa atttaaaatt gcgtatgtgg ttcacatcat    360
   acttttatta gaccacacta ctctaggaag agaaataatc actcattttc ttgtattact    420
   tctcattttc gtccatatca attcaagacg ttagatttcc tcctgcctgt cacagattct    480
   attagattta aaaccacagg gtcttttgct cttttctsta acttctacct tcaatatgca    540
   tttctcataa aatgtatctt tgcattttaa attcaataag cacatcaata tcaaaactgc    600
   ttttattctc ttcacttatc accccttcct attcaggaca ttactctttc ttttctccat    660
   cacaatttat tttttcctga catttttttc tggcttcctt ttcaaattag aaacctgc      718

## Description

### FIELD OF THE INVENTION

The present invention relates generally to the fields of genetics and medicine.

### BACKGROUND OF THE INVENTION

Approximately three to eight percent of the total health costs of modem industrialized countries are currently due to the direct costs of obesity (Wolf, 1996). In Germany, the total costs (both direct and indirect) related to obesity and comorbid disorders were estimated at 21 billion German marks (29.4 US Dollar) in 1995 (Schneider, 1996). By 2030 these costs will rise by 50% even if the prevalence of obesity does not increase further.

Obesity is often defined simply as a condition of abnormal or excessive fat accumulation in adipose tissue, to the extent that health may be impaired. The underlying disease is the process of undesirable positive energy balance and weight gain. An abdominal fat distribution is associated with higher health risks than a gynoid fat distribution.

The body mass index (BMI; kg/m²) provides the most useful, albeit crude, population-level measure of obesity. It can be used to estimate the prevalence of obesity within a population and the risks associated with it. However, BMI does not account for body compositon or body fat distribution (WHO, 1998).

**Table 1: Classification of overweight in adults according to BMI (WHO, 1998)**

| Classification | BMI (kg/m²) | Risk of co-morbidities |
|---|---|---|
| Underweight | < 18.5 | Low (but risks of other clinical problems increased) |
| Normal range | 18.5 - 24.9 | Average |
| Overweight | ≥ 25 | |
| Pre-obese | 25 - 29.9 | Increased |
| Obese class I | 30 - 34.9 | Moderate |
| Obese class II | 35 - 39.9 | Severe |
| Obese class III | ≥ 40 | Very severe |

Obesity has also been defined using the 85^{th} and 95^{th} BMI-percentiles as cutoffs for definition of obesity and severe obesity. BMI-percentiles have been calculated within several populations; centiles for the German population based on the German National Nutrition Survey have been available since 1994 (Hebebrand et al., 1994, 1996). Because the WHO classification of the different weight classes can only be applied to adults, it has become customary to refer to BMI-percentiles for the definition of obesity in children and adolescents.

The recent rise in the prevalence of obesity is an issue of major concern for the health systems of several countries. According to reports of the Center of Disease Control and Prevention (CDC) there has been a dramatic increase in obesity in the United States during the past 20 years. In 1985 only a few states were participating in CDC's Behavioral Risk Factor Surveillance System (BRFSS) and providing obesity data. In 1991, four states were reporting obesity prevalence rates of 15-19 percent and no states reported rates at or above 20 percent. In 2002, 20 states have obesity prevalence rates of 15-19 percent; 29 states have rates of 20-24 percent; and one state reports a rate over 25 percent. Similar trends have been observed in other countries in Europe and South America.

Children and adolescents have not been exempt from this trend. Quite to the contrary, the increase in the USA has been substantial. Thus, between the 1960ies and 1990, overweight and obesity increased dramatically in 6 through to 17 year olds. The increments translate into relative increases of 40% using the 85^{th} BMI-centile (calculated in the 1960ies) as a cutoff and 100% upon use of the 95^{th} centile. In a cross sectional study of German children and adolescents treated as inpatients for extreme obesity between 1985 and 1995, a significant increase of the mean BMI of almost 2 kg/m² over this ten year period has been reported. Within this extreme group, the increments were most pronounced in the uppermost BMI ranges.

The mechanisms underlying this increase in the prevalence of obesity are unknown. Environmental factors have commonly been invoked as the underlying cause. Basically, both an increased caloric intake and a reduced level of physical activity have been discussed. In England the increase in obesity rates has been attributed to the latter mechanism. Thus, in this country, the average caloric intake even decreased somewhat within the last two decades, whereas indirect evidence stemming from the increases in hours spent watching television and from the average number of cars per household points to reduced levels of physical activity as the relevant causative factor.

Genetic factors have previously not been considered as a contributing cause. Quite to the contrary, the fact that the increased rates of obesity have been observed within the last two decades has been viewed as evidence that genetic factors cannot be held responsible. However, it has been proposed that an increase in the rate of assortative mating could very well constitute a genetic contribution to the observed phenomenon. This hypothesis is based on evidence suggesting that stigmatisation of obese individuals represents a rather recent social phenomenon, thus invariably having led to increased rates of assortative mating. As a consequence, the offspring have a higher loading with both additive and non-additive genetic factors underlying obesity. Indeed, an exceedingly high rate of (deduced) assortative mating amongst the parents of extremely obese children and adolescents has been observed.

Potentially life-threatening, chronic health problems associated with obesity fall into four main areas: 1) cardiovascular problems, including hypertension, chronic heart disease and stroke, 2) conditions associated with insulin resistance, namely Non-Insulin Dependent Diabetes Mellitus (NIDDM), 3) certain types of cancers, mainly the hormonally related and large-bowel cancers, and 4) gallbladder disease. Other problems associated with obesity include respiratory difficulties, chronic musculo-skeletal problems, skin problems and infertility (WHO, 1998).

The main currently available strategies for treating these disorders include dietary restriction, increments in physical activity, pharmacological and surgical approaches. In adults, long term weight loss is exceptional using conservative interventions. Present pharmacological interventions typically induce a weight loss of between five and fifteen kilograms; if the medication is discontinued, renewed weight gain ensues. Surgical treatments are comparatively successful and are reserved for patients with extreme obesity and/or with serious medical complications.

Recently, a 10 year old massively obese girl, in whom a leptin deficiency mutation had been detected, was treated successfully with recombinant leptin. This is the first individual who therapeutically profited from the detection of the mutation underlying her morbid obesity.

Several twin studies have been performed to estimate heritability of the BMI, some of which have encompassed over 1000 twin pairs. The results have been very consistent: The intrapair correlations among monozygotic twins were typically between 0.6 and 0.8, independent of age and gender. In one study, the correlations for monozygotic and dizygotic twins were basically the same, independent of whether the twins had been reared apart or together. Heritability of the BMI was estimated at 0.7; non-shared environmental factors explained the remaining 30% of the variance. Surprisingly, shared environmental factors did not explain a substantial proportion of the variance. Both hypercaloric and hypocaloric alimentation lead to similar degrees of weight gain or loss among both members of monozygotic twin pairs, indicating that genetic factors regulate the effect of environmentally induced variation of energy availability on body weight. Metabolic reactions and changes in body fat distribution upon overeating and undereating are also under genetic control (reviewed in Hebebrand et al., 1998).

A large adoption study has revealed that the BMI of adoptees is correlated with that of their biological parents and not with the BMI of the adoptive parents. Depending on the family study, the correlation between the BMI of sibs is between 0.2 and 0.4. Parent-offspring correlations are typically slightly lower. Segregation analyses have repeatedly suggested a major recessive gene effect. Based on these analyses, sample size calculations have been performed based on both concordant and discordant approaches. In contrast to the expectations, the concordant sib-pair approach was superior; a lower number of families were required to achieve the same power.

Family studies based on extremely obese young index patients, either mother or father or both, have a BMI > 90^{th} decile in the vast majority of the families. Based on index patients with a BMI > 95^{th} centile, approximately 20% of the respective families have a sib with a BMI > 90^{th} centile.

In conclusion, it is apparent that environmental factors interact with specific genotypes rendering an individual more or less susceptible to the development of obesity. Furthermore, despite the fact that major genes have been detected, it is necessary to consider that the spectrum reaches from such major genes to genes with an only minor influence.

The discovery of the leptin gene at the end of 1994 (Zhang et al., 1994) has been followed by a virtual explosion of scientific efforts to uncover the regulatory systems underlying appetite and weight regulation. It is currently the fastest growing biomedical field. This upswing has also resulted in large scaled molecular genetic activities which, due to obvious clinical interest, are basically all related to obesity in humans, rodents and other mammals (Hebebrand et al., 1998).

In this respect, many genes in which mutations lead to the presently known monogenic forms of obesity have been cloned in rodents. Systemic consequences of these mutations are currently being analysed. These models have provided insights into the complex regulatory systems involved in body weight regulation, the best known of which includes leptin and its receptor.

In mice, but also in pigs, over 15 quantitative trait loci (QTL) have been identified that are most likely relevant in weight regulation (Chagnon et al., 2003).

In humans, four exceedingly rare autosomal recessive forms of obesity have been described as of 1997. Mutations in the genes encoding for leptin, leptin receptor, prohormone convertase 1 and pro-opiomelanocortin (POMC) have been shown to cause massive obesity of an early onset type, associated with hyperphagia. Distinct additional clinical (e.g. red hair, primary amenorrhea) and/or endocrinological abnormalities (e.g. markedly altered serum leptin levels, lack of ACTH secretion) pinpointed to the respective candidate genes. Both the monogenic animal models and the human monogenic forms have led to new insights into the complex system underlying body weight regulation.

Very recently, the first autosomal dominant form of obesity was described in humans. Two different mutations within the melanocortin-4 receptor gene (*MC4R*) were observed to lead to extreme obesity in probands heterozygous for these variants. In contrast to the aforementioned findings, these mutations do not implicate readily obvious phenotypic abnormalities other than extreme obesity (Vaisse et al., 1998; Yeo et al., 1998). Interestingly, both groups detected the mutations by systematic screens in relatively small study groups (n=63 and n=43).

Hinney et al. (1999) screened the *MC4R* in a total of 492 obese children and adolescents. All in all, four individuals with two different mutations leading to haplo-insufficiency were detected. One was identical to that previously observed by Yeo et al. (1998). The other mutation, which was detected in three individuals, induced a stop mutation in the extracellular domain of the receptor. Approximately one percent of extremely obese individuals harbour haplo-insuffciency mutations in the *MC4R*. In addition to the two forms of haplo-insuffciency, Hinney et al. (1999) also detected additional mutations leading to both conservative and non-conservative amino acid exchanges. Interestingly, these mutations were mainly observed in the obese study group. The functional implications of these mutations are currently unknown.

The identification of individuals with *MC4R* mutations is interesting in the light of possible pharmacological interventions. Thus, intranasal application of adrenocorticotropin₄₋₁₀ (ACTH₄₋₁₀), representing a core sequence of all melanocortins, resulted in reduced weight, body fat mass and plasma leptin concentrations in healthy controls. The question arises as to how mutation carriers would react to this treatment, which could theoretically counterbalance their reduced receptor density.

The involvement of specific genes in weight regulation is further substantiated by data obtained from transgenic mice. For example, MC4R deficient mice develop early onset obesity (Huszar et al., 1997).

EP-A-1 403 380 (Integragen) discloses PPYR1 as a human obesity susceptibility marker.

Different groups are conducting genome scans related to obesity or dependent phenotypes (BMI, leptin levels, fat mass, etc.). This approach appears very promising, because it is both systematic and model free. In addition, it has already been shown to be exceptionally successful. Thus, positive linkage results have been obtained even by analysing comparatively small study groups. More important, some findings have already been replicated. Each of the following regions has been identified by at least two independent groups: chromosome 1p32, chromosome 2p21, chromosome 6p21, chromosome 10 and chromosome 20q13 (Chagnon et al., 2003).

### SUMMARY

The present invention now discloses the identification of a human obesity susceptibility gene, which can be used for the diagnosis, prevention and treatment of obesity, as well as for the screening of therapeutically active drugs.

The present invention more particularly discloses the identification of a human obesity susceptibility gene, which can be used for the diagnosis, prevention and treatment of obesity, as well as for the screening of therapeutically active drugs. The invention more specifically discloses certain alleles of the contactin associated protein-like 2 (CNTNAP2) gene related to susceptibility to obesity and representing novel targets for therapeutic intervention. The present invention relates to particular mutations in the CNTNAP2 gene and expression products, as well as to diagnostic tools and kits based on these mutations. The diagnosis of predisposition to, detection, prevention and/or treatment of coronary heart disease and metabolic disorders, including but not limited to hypoalphalipoproteinemia, familial combined hyperlipidemia, insulin resistant syndrome X or multiple metabolic disorder, coronary artery disease, diabetes and associated complications and dyslipidemia, is described.

The diagnosis of predisposition to or protection from, detection, prevention and/or treatment of obesity or associated disorders is described, the method comprising detecting in a sample from the subject the presence of an alteration in the CNTNAP2 gene or polypeptide, the presence of said alteration being indicative of the presence or predisposition to obesity or associated disorders. The presence of said alteration can also be indicative for protecting against obesity.

A particular object resides in a method of detecting the presence of or predisposition to obesity or associated disorders in a subject, the method comprising detecting the presence of an alteration in the CNTNAP2 gene locus in a sample from the subject, the presence of said alteration being indicative of the presence of or the predisposition to obesity or associated disorders.

An additional particular object resides in a method of detecting the protection from obesity or associated disorders in a subject, the method comprising detecting the presence of an alteration in the CNTNAP2 gene locus in a sample from the subject, the presence of said alteration being indicative of the protection from obesity or associated disorders.

Another particular object resides in a method of assessing the response of a subject to a treatment of obesity or associated disorders, the method comprising detecting the presence of an alteration in the CNTNAP2 gene locus in a sample from the subject, the presence of said alteration being indicative of a particular response to said treatment.

A further particular object resides in a method of assessing the adverse effect in a subject to a treatment of obesity or associated disorders, the method comprising detecting the presence of an alteration in the CNTNAP2 gene locus in a sample from the subject, the presence of said alteration being indicative of an adverse effect to said treatment.

A method for preventing obesity or an associated disorder in a subject is described, comprising detecting the presence of an alteration in the CNTNAP2 gene locus in a sample from the subject, the presence of said alteration being indicative of the predisposition to obesity or associated disorders; and, administering a prophylactic treatment against obesity or an associated disorder.

In a preferred embodiment, said alteration is one or several SNP(s) or a haplotype of SNPs associated with obesity or an associated disorder. More preferably, said haplotype associated with obesity or an associated disorder comprises or consists of several SNPs selected from the group consisting of SNP 11, SNP 14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 and SNP163. Still more preferably, said haplotype is selected from the haplotypes disclosed in Table 4. More preferably, said SNP associated with obesity or an associated disorder can be selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. More preferably, said SNP associated with protection against obesity can be selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. In one particularly preferred embodiment, said SNP associated with protection against obesity can be SNP156, more particularly allele 2 of this SNP.

Preferably, the alteration in the CNTNAP2 gene locus is determined by performing a hydridization assay, a sequencing assay, a microsequencing assay, or an allele-specific amplification assay.

A particular aspect resides in compositions of matter comprising primers, probes, and/or oligonucleotides, which are designed to specifically detect at least one SNP or haplotype associated with obesity in the genomic region including the CNTNAP2 gene, or a combination thereof. More preferably, said haplotype associated with obesity or an associated disorder comprises or consists of several SNPs selected from the group consisting of SNP11, SNP14, SNP40, SNP45, SNP47, SNP48, SNP49, SNP50, SNP51, SNP53, SNP56, SNP60, SNP63, SNP65, SNP77, SNP94, SNF111, SNP112, SNP113, SNP116, SNP141, SNP142, SNP156, SNP161 and SNP163. Still more preferably, said haplotype is selected from the haplotypes disclosed in Table 4. More preferably, said SNP associated with obesity or an associated disorder can be selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. More preferably, said SNP associated with protection against obesity can be selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. In one particularly preferred embodiment, said SNP associated with protection against obesity can be SNP156, more particularly allele 2 of this SNP.

Methods of treating obesity and/or associated disorders in a subject through a modulation of CNTNAP2 expression or activity are described. Such treatments use, for instance, CNTNAP2 polypeptides, CNTNAP2 DNA sequences (including antisense sequences and RNAi directed at the CNTNAP2 gene locus), anti- CNTNAP2 antibodies or drugs that modulate CNTNAP2 expression or activity.

Methods of treating individuals who carry deleterious alleles of the CNTNAP2 gene, including pre-symptomatic treatment or combined therapy, such as through gene therapy, protein replacement therapy or through the administration of CNTNAP2 protein mimetics and/or inhibitors, are also described.

A further aspect resides in the screening of drugs for therapy of obesity or associated disorder, based on the modulation of or binding to an allele of CNTNAP2 gene associated with obesity or associated disorder or gene product thereof.

A further aspect includes antibodies specific of CNTNAP2 polypeptide fragments and derivatives of such antibodies, hybridomas secreting such antibodies, and diagnostic kits comprising those antibodies. More preferably, said antibodies are specific to a CNTNAP2 polypeptide or a fragment thereof comprising an alteration, said alteration modifying the activity of CNTNAP2.

A CNTNAP2 gene or a fragment thereof comprising an alteration, said alteration modifying the activity of CNTNAP2, is also described. A CNTNAP2 polypeptide or a fragment thereof comprising an alteration, said alteration modifying the activity of CNTNAP2, is also described.

### LEGEND TO THE FIGURES

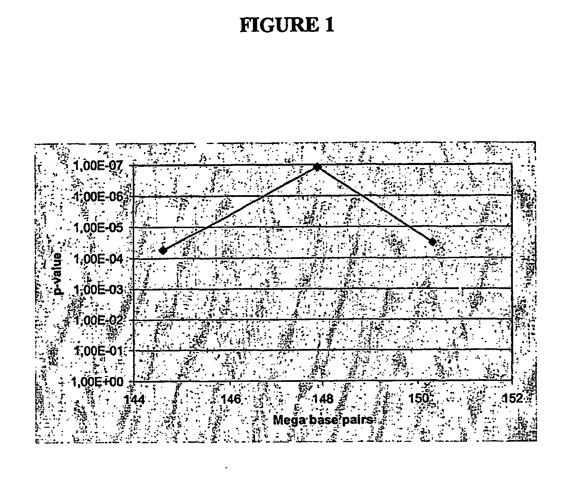

### Figure 1 : High density mapping using Genomic Hybrid Identity Profiling (GenomeHIP)

A total of 2263 BAC clones with an average spacing of 1.2 Mega base pairs between clones representing the whole human genome were tested for linkage using GenomeHIP. Each point corresponds to a clone. Significant evidence for linkage was calculated for clone BACA2ZB02 (p-value 1.20E-07). The whole linkage region encompasses a region from 4 144 736 618 base pairs to 150 314 820 base pairs on human chromosome 7. The p-value 2x10⁻⁵ corresponding to the significance level for significant linkage was used as a significance level for whole genome screens as proposed by Lander and Kruglyak (1995).

### DETAILED DESCRIPTION OF THE INVENTION

The present invention discloses the identification of CNTNAP2 as a human obesity susceptibility gene. Various nucleic acid samples from families with obesity were submitted to a particular GenomeHIP process. This process led to the identification of particular identical-by-descent fragments in said populations that are altered in obese subjects. By screening of the IBD fragments, we identified the contactin associated protein-like 2 gene on chromosome 7q35-q36 (CNTNAP2) as a candidate for obesity and related disorders. This gene is indeed present in the critical interval and expresses a functional phenotype consistent with a genetic regulation of obesity. SNPs of the CNTNAP2 gene were also identified, as being correlated to obesity in human subjects. SNP45, SNP47, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163, located in the CNTNAP2 gene locus were found to be associated with obesity or an associated disorder. Haplotypes disclosed in Table 4 comprising several SNPs selected from the group consisting of SNP11, SNP14, SNP40, SNP45, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 and SNP163 have also been identified as associated with obesity. SNP156, more particularly allele 2, was also found to be associated with a protection against obesity.

It is thus proposed to use CNTNAP2 gene and corresponding expression products for the diagnosis, prevention and treatment of obesity and associated disorders, as well as for the screening of therapeutically active drugs.

### DEFINITIONS

Obesity and metabolic disorders: Obesity shall be construed as any condition of abnormal or excessive fat accumulation in adipose tissue, to the extent that health may be impaired. Associated disorders, diseases or pathologies include, more specifically, any metabolic disorders, including diabetes mellitus (more particularly type II diabetes) and associated complications such as diabetic neuropathy, hypo-alphalipoproteinemia, familial combined hyperlipidemia, hyperinsulinemia, insulin resistance, insulin resistant syndrome X or multiple metabolic disorder, cardiovascular complications such as coronary artery disease, and dyslipidemia. Preferred associated disorders are selected from the group consisting of type II diabetes, hyperinsulinemia, insulin resistance, and diabetic neuropathy.

The invention may be used in various subjects, particularly human, including adults, children and at the prenatal stage.

Within the context of this invention, the CNTNAP2 gene locus designates all CNTNAP2 sequences or products in a cell or organism, including CNTNAP2 coding sequences, CNTNAP2 non-coding sequences (e.g., introns), CNTNAP2 regulatory sequences controlling transcription and/or translation (e.g., promoter, enhancer, terminator, etc.), as well as all corresponding expression products, such as CNTNAP2 RNAs (e.g., mRNAs) and CNTNAP2 polypeptides (e.g., a pre-protein and a mature protein). The CNTNAP2 gene locus also comprise surrounding sequences of the CNTNAP2 gene which include SNPs that are in linkage disequilibrium with SNPs located in the CNTNAP2 gene. For example, the CNTNAP2 locus comprises surrounding sequences comprising SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP 113, SNP116, SNP141, SNP142, SNP156, SNP161 and SNP163.

As used in the present application, the term "CNTNAP2 gene" designates the contactin associated protein-like 2 gene on chromosome 7q35-q36, as well as variants, analogs and fragments thereof, including alleles thereof (e.g., germline mutations) which are related to susceptibility to obesity and associated disorders. The CNTNAP2 gene may also be referred to as contactin-associated protein 2, cell recognition molecule (CASPR2), homolog of Drosophilia neurexin IV (NRXN4).

The term "gene" shall be construed to include any type of coding nucleic acid, including genomic DNA (gDNA), complementary DNA (cDNA), synthetic or semi-synthetic DNA, as well as any form of corresponding RNA. The term gene particularly includes recombinant nucleic acids encoding CNTNAP2, i.e., any non naturally occurring nucleic acid molecule created artificially, e.g., by assembling, cutting, ligating or amplifying sequences. A CNTNAP2 gene is typically double-stranded, although other forms may be contemplated, such as single-stranded. CNTNAP2 genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable CNTNAP2 gene sequences may be found on gene banks, such as Unigene Cluster for CNTNAP2 (Hs.106552) and Unigene Representative Sequence NM_014141. A particular example of a CNTNAP2 gene comprises SEQ ID No: 1.

The term "CNTNAP2 gene" includes any variant, fragment or analog of SEQ ID No 1 or of any coding sequence as identified above. Such variants include, for instance, naturally-occurring variants due to allelic variations between individuals (e.g., polymorphisms), mutated alleles related to obesity or an associated disorder, alternative splicing forms, etc. The term variant also includes CNTNAP2 gene sequences from other sources or organisms. Variants are preferably substantially homologous to SEQ ID No 1, i.e., exhibit a nucleotide sequence identity of at least about 65%, typically at least about 75%, preferably at least about 85%, more preferably at least about 95% with SEQ ID No 1. Variants and analogs of a CNTNAP2 gene also include nucleic acid sequences, which hybridize to a sequence as defined above (or a complementary strand thereof) under stringent hybridization conditions.

Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of a CNTNAP2 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide lengths between 8 and 100 nucleotides, preferably between 15 and 100, more preferably between 20 and 100.

A CNTNAP2 polypeptide designates any protein or polypeptide encoded by a CNTNAP2 gene as disclosed above. The term "polypeptide" refers to any molecule comprising a stretch of amino acids. This term includes molecules of various lengths, such as peptides and proteins. The polypeptide may be modified, such as by glycosylations and/or acetylations and/or chemical reaction or coupling, and may contain one or several non-natural or synthetic amino acids. A specific example of a CNTNAP2 polypeptide comprises all or part of SEQ ID No: 2 (NP_054860).

The terms "response to a treatment" refer to treatment efficacy, including but not limited to ability to metabolise a therapeutic compound, to the ability to convert a pro-drug to an active drug, and to the pharmacokinetics (absorption, distribution, elimination) and the pharmacodynamics (receptor-related) of a drug in an individual.

The terms "adverse effects to a treatment" refer to adverse effects of therapy resulting from extensions of the principal pharmacological action of the drug or to idiosyncratic adverse reactions resulting from an interaction of the drug with unique host factors. "Side effects to a treatment" include, but are not limited to, adverse reactions such as dermatologic, hematologic or hepatologic toxicities and further includes gastric and intestinal ulceration, disturbance in platelet function, renal injury, generalized urticaria, bronchoconstriction, hypotension, and shock.

### DIAGNOSIS

Diagnosis methods based on a monitoring of the CNTNAP2 gene locus in a subject are provided. Within the context of the present invention, the term 'diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmacogenetics), etc.

Diagnostic methods to determine whether an individual is at risk of developing obesity or an obesity-associated disorder or suffers from obesity or an obesity-associated disorder resulting from a mutation or a polymorphism in the CNTNAP2 gene locus are provided. Methods to determine whether an individual is likely to respond positively to a therapeutic agent or whether an individual is at risk of developing an adverse side effect to a therapeutic agent, are also provided.

A particular object resides in a method of detecting the presence of or predisposition to obesity or an obesity-associated disorder in a subject, the method comprising detecting in a sample from the subject the presence of an alteration in the CNTNAP2 gene locus in said sample. The presence of said alteration is indicative of the presence or predisposition to obesity or an obesity-associated disorder. Optionally, said method comprises a previous step of providing a sample from a subject. Preferably, the presence of an alteration in the CNTNAP2 gene locus in said sample is detected through the genotyping of a sample.

Another particular object resides in a method of detecting the protection from obesity or an obesity-associated disorder in a subject, the method comprising detecting the presence of an alteration in the CNTNAP2 gene locus in a sample from the subject, the presence of said alteration being indicative of the protection from obesity or an obesity-associated disorder.

In a preferred embodiment, said alteration is one or several SNP(s) or a haplotype of SNPs associated with obesity or an associated disorder. More preferably, said haplotype associated with obesity or an associated disorder comprises or consists of several SNPs selected from the group consisting of SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 and SNP163. Still more preferably, said haplotype is selected from the haplotypes disclosed in Table 4. More preferably, said SNP associated with obesity or an associated disorder is selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. More preferably, said SNP associated with protection against obesity can be selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. In one particularly preferred embodiment, said SNP associated with protection against obesity can be SNP156, more particularly allele 2 of this SNP.

Another particular object resides in a method of assessing the response of a subject to a treatment of obesity or an associated disorder, the method comprising (i) providing a sample from the subject and (ii) detecting the presence of an alteration in the CNTNAP2 gene locus in said sample.

Another particular object resides in a method of assessing the response of a subject to a treatment of obesity or an associated disorder, the method comprising detecting in a sample from the subject the presence of an alteration in the CNTNAP2 gene locus in said sample. The presence of said alteration is indicative of a particular response to said treatment. Preferably, the presence of an alteration in the CNTNAP2 gene locus in said sample is detected through the genotyping of a sample.

A further particular object resides in a method of assessing the adverse effects of a subject to a treatment of obesity or an associated disorder, the method comprising detecting in a sample from the subject the presence of an alteration in the CNTNAP2 gene locus in said sample. The presence of said alteration is indicative of adverse effects to said treatment. Preferably, the presence of an alteration in the CNTNAP2 gene locus in said sample is detected through the genotyping of a sample.

In a preferred embodiment, said alteration is one or several SNP(s) or a haplotype of SNPs associated with obesity or an associated disorder. More preferably, said haplotype associated with obesity or an associated disorder comprises or consists of several SNPs selected from the group consisting of SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 and SNP163. Still more preferably, said haplotype is selected from the haplotypes disclosed in Table 4. More preferably, said SNP associated with obesity or an associated disorder is selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. More preferably, said SNP associated with protection against obesity can be selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, SNP161 and SNP163. In one particularly preferred embodiment, said SNP associated with protection against obesity can be SNP156, more particularly allele 2 of this SNP.

In an additional embodiment, a method for preventing obesity or an associated disorder in a subject is provided, comprising detecting the presence of an alteration in the CNTNAP2 gene locus in a sample from the subject, the presence of said alteration being indicative of the predisposition to obesity or an associated disorder; and, administering a prophylactic treatment against obesity or an associated disorder. Said prophylactic treatment can be a drug administration and/or a diet.

Diagnostics, which analyse and predict response to a treatment or drug, or side effects to a treatment or drug, may be used to determine whether an individual should be treated with a particular treatment drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

Clinical drug trials represent another application for the CNTNAP2 SNPs. One or more CNTNAP2 SNPs indicative of response to a drug or to side effects to a drug may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

The alteration may be determined at the level of the CNTNAP2 gDNA, RNA or polypeptide. Optionally, the detection is performed by sequencing all or part of the CNTNAP2 gene or by selective hybridisation or amplification of all or part of the CNTNAP2 gene. More preferably a CNTNAP2 gene specific amplification is carried out before the alteration identification step.

An alteration in the CNTNAP2 gene locus may be any form of mutation(s), deletion(s), rearrangement(s) and/or insertions in the coding and/or non-coding region of the locus, alone or in various combination(s). Mutations more specifically include point mutations. Deletions may encompass any region of two or more residues in a coding or non-coding portion of the gene locus, such as from two residues up to the entire gene or locus. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene locus. Insertions may typically comprise an addition of between 1 and 50 base pairs in the gene locus. Rearrangement includes inversion of sequences. The CNTNAP2 gene locus alteration may result in the creation of stop codons, frameshift mutations, amino acid substitutions, particular RNA splicing or processing, product instability, truncated polypeptide production, etc. The alteration may result in the production of a CNTNAP2 polypeptide with altered function, stability, targeting or structure. The alteration may also cause a reduction in protein expression or, alternatively, an increase in said production.

In a particular embodiment of the method disclosed, the alteration in the CNTNAP2 gene locus is selected from a point mutation, a deletion and an insertion in the CNTNAP2 gene or corresponding expression product, more preferably a point mutation and a deletion. The alteration may be determined at the level of the CNTNAP2 gDNA, RNA or polypeptide.

In this regard, SNPs in the CNTNAP2 gene and certain haplotypes, which include SNPs selected from the group consisting of SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 and SNP163, that are associated with obesity or an associated disorder, are now disclosed. The SNPs are reported in the following Table 1.

**Table 1**

| Position in chrom 7 based on NCBI Build 34 | SNP identity | dbSNP reference | Allele 1 | Allele 2 | Position in locus and type of amino acid change | SEQ ID No |
|---|---|---|---|---|---|---|
| 143467490 | 11 | rs1635079 | A | G | 5' of CNTNAP2 | 3 |
| 143543795 | 14 | rs886469 | C | T | 5' of CNTNAP2 | 4 |
| 145272638 | 40 | rs12703780 | A | G | intron | 5 |
| 145367704 | 45 | rs1405109 | C | T | intron | 6 |
| 145399533 | 47 | rs7806058 | A | G | intron | 7 |
| 145424265 | 48 | rs7788551 | C | T | intron | 8 |
| 145446406 | 49 | rs7809716 | A | G | intron | 9 |
| 145456858 | 50 | rs6464757 | C | T | intron | 10 |
| 145484824 | 51 | rs1639481 | A | G | intron | 11 |
| 145519990 | 53 | rs7784672 | A | G | intron | 12 |
| 145568121 | 56 | rs700273 | G | T | intron | 13 |
| 145654078 | 60 | rs1253S870 | A | G | intron | 14 |
| 145709388 | 63 | rs1155384 | C | T | intron | 15 |
| 145734063 | 65 | rs7789802 | A | C | intron | 16 |
| 146043201 | 77 | rs13221567 | A | C | intron | 17 |
| 146312130 | 94 | Not available | C | G | intron | 18 |
| 146603365 | 111 | rs851840 | C | G | intron | 19 |
| 146609747 | 112 | rs2888493 | A | G | intron | 20 |
| 146617641 | 113 | rs11764315 | A | C | intron | 21 |
| 146668312 | 116 | rs989242 | A | G | intron | 22 |
| 147225657 | 141 | rs2972125 | C | T | intron | 23 |
| 147254373 | 142 | rs963314 | A | G | intron | 24 |
| 147436213 | 156 | rs10278502 | C | T | intron | 25 |
| 147503860 | 161 | rs2270069 | C | T | intron | 26 |
| 147544244 | 163 | rs2717775 | C | G | 3' ofCNTNAP2 | 27 |

In any method disclosed herein, one or several SNP in the CNTNAP2 gene and certain haplotypes comprising SNP in the CNTNAP2 gene, more particularly SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 and SNP163, can be used in combination with other SNP or haplotype associated with obesity or an associated disorder and located in other gene(s).

In another variant, the method comprises detecting the presence of an altered CNTNAP2 RNA expression. Altered RNA expression includes the presence of an altered RNA sequence, the presence of an altered RNA splicing or processing, the presence of an altered quantity of RNA, etc. These may be detected by various techniques known in the art, including by sequencing all or part of the CNTNAP2 RNA or by selective hybridisation or selective amplification of all or part of said RNA, for instance.

In a further variant, the method comprises detecting the presence of an altered CNTNAP2 polypeptide expression. Altered CNTNAP2 polypeptide expression includes the presence of an altered polypeptide sequence, the presence of an altered quantity of CNTNAP2 polypeptide, the presence of an altered tissue distribution, etc. These may be detected by various techniques known in the art, including by sequencing and/or binding to specific ligands (such as antibodies), for instance.

As indicated above, various techniques known in the art may be used to detect or quantify altered CNTNAP2 gene or RNA expression or sequence, including sequencing, hybridisation, amplification and/or binding to specific ligands (such as antibodies). Other suitable methods include allele-specific oligonucleotide (ASO), allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, heteroduplex analysis, RNase protection, chemical mismatch cleavage, ELISA, radio-immunoassays (RIA) and immuno-enzymatic assays (IEMA).

Some of these approaches (e.g., SSCA and CGGE) are based on a change in electrophoretic mobility of the nucleic acids, as a result of the presence of an altered sequence. According to these techniques, the altered sequence is visualized by a shift in mobility on gels. The fragments may then be sequenced to confirm the alteration.

Some others are based on specific hybridisation between nucleic acids from the subject and a probe specific for wild type or altered CNTNAP2 gene or RNA. The probe may be in suspension or immobilized on a substrate. The probe is typically labeled to facilitate detection of hybrids.

Some of these approaches are particularly suited for assessing a polypeptide sequence or expression level, such as Northern blot, ELISA and RIA. These latter require the use of a ligand specific for the polypeptide, more preferably of a specific antibody.

In a particular, preferred, embodiment, the method comprises detecting the presence of an altered CNTNAP2 gene expression profile in a sample from the subject. As indicated above, this can be accomplished more preferably by sequencing, selective hybridisation and/or selective amplification of nucleic acids present in said sample.

### Sequencing

Sequencing can be carried out using techniques well known in the art, using automatic sequencers. The sequencing may be performed on the complete CNTNAP2 gene or, more preferably, on specific domains thereof, typically those known or suspected to carry deleterious mutations or other alterations.

### Amplification

Amplification is based on the formation of specific hybrids between complementary nucleic acid sequences that serve to initiate nucleic acid reproduction.

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the CNTNAP2 gene or locus are able to specifically hybridize with a portion of the CNTNAP2 gene locus that flank a target region of said locus, said target region being altered in certain subjects having obesity or an associated disorder. Examples of such target regions are provided in Table 1.

Primers that can be used to amplify CNTNAP2 target region comprising SNPs as identified in Table 1 may be designed based on the sequence of Seq Id No 1 or on the genomic sequence of CNTNAP2. In a particular embodiment, primers may be designed based on the sequence of SEQ ID Nos 3-27.

Another particular object resides in a nucleic acid primer useful for amplifying sequences from the CNTNAP2 gene or locus including surrounding regions. Such primers are preferably complementary to, and hybridize specifically to nucleic acid sequences in the CNTNAP2 gene locus. Particular primers are able to specifically hybridise with a portion of the CNTNAP2 gene locus that flank a target region of said locus, said target region being altered in certain subjects having obesity or an associated disorder.

A nucleic acid primer is provided, said primer being complementary to and hybridizing specifically to a portion of a CNTNAP2 coding sequence (e.g., gene or RNA) altered in certain subjects having obesity or an associated disorder. In this regard, particular primers described herein are specific for altered sequences in a CNTNAP2 gene or RNA. By using such primers, the detection of an amplification product indicates the presence of an alteration in the CNTNAP2 gene locus. In contrast, the absence of amplification product indicates that the specific alteration is not present in the sample.

Typical primers are single-stranded nucleic acid molecules of about 5 to 60 nucleotides in length, more preferably of about 8 to about 25 nucleotides in length. The sequence can be derived directly from the sequence of the CNTNAP2 gene locus. Perfect complementarity is preferred, to ensure high specificity. However, certain mismatch may be tolerated.

The use of a nucleic acid primer or a pair of nucleic acid primers as described above in a method of detecting the presence of or predisposition to obesity or an associated disorder in a subject or in a method of assessing the response of a subject to a treatment of obesity or an associated disorder, is also described.

### Selective hybridization

Hybridization detection methods are based on the formation of specific hybrids between complementary nucleic acid sequences that serve to detect nucleic acid sequence alteration(s).

A particular detection technique involves the use of a nucleic acid probe specific for wild type or altered CNTNAP2 gene or RNA, followed by the detection of the presence of a hybrid. The probe may be in suspension or immobilized on a substrate or support (as in nucleic acid array or chips technologies). The probe is typically labeled to facilitate detection of hybrids.

In this regard, a particular embodiment comprises contacting the sample from the subject with a nucleic acid probe specific for an altered CNTNAP2 gene locus, and assessing the formation of an hybrid. In a particular, preferred embodiment, the method comprises contacting simultaneously the sample with a set of probes that are specific, respectively, for wild type CNTNAP2 gene locus and for various altered forms thereof. In this embodiment, it is possible to detect directly the presence of various forms of alterations in the CNTNAP2 gene locus in the sample. Also, various samples from various subjects may be treated in parallel.

Within the present context, a probe refers to a polynucleotide sequence which is complementary to and capable of specific hybridisation with a (target portion of a) CNTNAP2 gene or RNA, and which is suitable for detecting polynucleotide polymorphisms associated with CNTNAP2 alleles which predispose to or are associated with obesity or an associated disorder. Probes are preferably perfectly complementary to the CNTNAP2 gene, RNA, or target portion thereof. Probes typically comprise single-stranded nucleic acids of between 8 to 1000 nucleotides in length, for instance of between 10 and 800, more preferably of between 15 and 700, typically of between 20 and 500. It should be understood that longer probes may be used as well. A preferred probe of this invention is a single stranded nucleic acid molecule of between 8 to 500 nucleotides in length, which can specifically hybridise to a region of a CNTNAP2 gene or RNA that carries an alteration.

A specific embodiment is a nucleic acid probe specific for an altered (e.g., a mutated) CNTNAP2 gene or RNA, i.e., a nucleic acid probe that specifically hybridises to said altered CNTNAP2 gene or RNA and essentially does not hybridise to a CNTNAP2 gene or RNA lacking said alteration. Specificity indicates that hybridisation to the target sequence generates a specific signal which can be distinguished from the signal generated through non-specific hybridisation. Perfectly complementary sequences are preferred to design probes according to this invention. It should be understood, however, that certain a certain degree of mismatch may be tolerated, as long as the specific signal may be distinguished from non-specific hybridisation.

Particular examples of such probes are nucleic acid sequences complementary to a target portion of the genomic region including the CNTNAP2 gene or RNA carrying a point mutation as listed in Table 1 above. More particularly, the probes can comprise a sequence selected from the group consisting of SEQ ID Nos 3-27 or a fragment thereof comprising the SNP or a complementary sequence thereof.

The sequence of the probes can be derived from the sequences of the CNTNAP2 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labeling, etc.

The use of a nucleic acid probe as described above in a method of detecting the presence of or predisposition to obesity or an associated disorder in a subject or in a method of assessing the response of a subject to a treatment of obesity or an associated disorder, is also described.

### Specific Ligand Binding

As indicated above, alteration in the CNTNAP2 gene locus may also be detected by screening for alteration(s) in CNTNAP2 polypeptide sequence or expression levels. In this regard, a specific embodiment of this invention comprises contacting the sample with a ligand specific for a CNTNAP2 polypeptide and determining the formation of a complex.

Different types of ligands may be used, such as specific antibodies. In a specific embodiment, the sample is contacted with an antibody specific for a CNTNAP2 polypeptide and the formation of an immune complex is determined. Various methods for detecting an immune complex can be used, such as ELISA, radioimmunoassays (RIA) and immuno-enzymatic assays (IEMA).

Within the present context, an antibody designates a polyclonal antibody, a monoclonal antibody, as well as fragments or derivatives thereof having substantially the same antigen specificity. Fragments include Fab, Fab'2, CDR regions, etc. Derivatives include single-chain antibodies, humanized antibodies, poly functional antibodies, etc.

An antibody specific for a CNTNAP2 polypeptide designates an antibody that selectively binds a CNTNAP2 polypeptide, namely, an antibody raised against a CNTNAP2 polypeptide or an epitope-containing fragment thereof. Although non-specific binding towards other antigens may occur, binding to the target CNTNAP2 polypeptide occurs with a higher affinity and can be reliably discriminated from non-specific binding.

In a specific embodiment, the method comprises contacting a sample from the subject with (a support coated with) an antibody specific for an altered form of a CNTNAP2 polypeptide, and determining the presence of an immune complex. In a particular embodiment, the sample may be contacted simultaneously, or in parallel, or sequentially, with various (supports coated with) antibodies specific for different forms of a CNTNAP2 polypeptide, such as a wild type and various altered forms thereof.

The use of a ligand, preferably an antibody, a fragment or a derivative thereof as described above, in a method of detecting the presence of or predisposition to obesity or an associated disorder in a subject or in a method of assessing the response of a subject to a treatment of obesity or an associated disorder, is also described.

A diagnostic kit comprising products and reagents for detecting in a sample from a subject the presence of an alteration in the CNTNAP2 gene or polypeptide, in the CNTNAP2 gene or polypeptide expression, and/or in CNTNAP2 activity, is also described. Said diagnostic kit according comprises any primer, any pair of rimers, any nucleic acid probe and/or any ligand, preferably antibody, described herein. Said diagnostic kit can further comprise reagents and/or protocols for performing a hybridization, amplification or antigen-antibody immune reaction.

The diagnosis methods can be performed in vitro, ex vivo or in vivo, preferably in vitro or ex vivo. They use a sample from the subject, to assess the status of the CNTNAP2 gene locus. The sample may be any biological sample derived from a subject, which contains nucleic acids or polypeptides. Examples of such samples include fluids, tissues, cell samples, organs, biopsies, etc. Most preferred samples are blood, plasma, saliva, urine, seminal fluid, etc. Pre-natal diagnosis may also be performed by testing fetal cells or placental cells, for instance. The sample may be collected according to conventional techniques and used directly for diagnosis or stored. The sample may be treated prior to performing the method, in order to render or improve availability of nucleic acids or polypeptides for testing. Treatments include, for instant, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence of an altered CNTNAP2 gene locus. Contacting may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered CNTNAP2 polypeptide, RNA or DNA in the sample is indicative of the presence of an altered CNTNAP2 gene locus in the subject, which can be correlated to the presence, predisposition or stage of progression of obesity or an associated disorder. For example, an individual having a germ line CNTNAP2 mutation has an increased risk of developing obesity or an associated disorder. The determination of the presence of an altered CNTNAP2 gene locus in a subject also allows the design of appropriate therapeutic intervention, which is more effective and customized. Also, this determination at the pre-symptomatic level allows a preventive regimen to be applied.

### Linkage Disequilibirum

Once a first SNP has been identified in a genomic region of interest, more particularly in CNTNAP2 gene locus, the practitioner of ordinary skill in the art can easily identify additional SNPs in linkage disequilibrium with this first SNP. Indeed, any SNP in linkage disequilibrium with a first SNP associated with obesity or an associated disorder will be associated with this trait. Therefore, once the association has been demonstrated between a given SNP and obesity or an associated disorder, the discovery of additional SNPs associated with this trait can be of great interest in order to increase the density of SNPs in this particular region.

Identification of additional SNPs in linkage disequilibrium with a given SNP involves: (a) amplifying a fragment from the genomic region comprising or surrounding a first SNP from a plurality of individuals; (b) identifying of second SNPs in the genomic region harboring or surrounding said first SNP; (c) conducting a linkage disequilibrium analysis between said first SNP and second SNPs; and (d) selecting said second SNPs as being in linkage disequilibrium with said first marker. Subcombinations comprising steps (b) and (c) are also contemplated.

Methods to identify SNPs and to conduct linkage disequilibrium analysis can be carried out by the skilled person without undue experimentation by using well-known methods.

These SNPs in linkage disequilibrium can also be used in the methods described herein, and more particularly in the diagnosic methods described herein.

For example, a linkage locus of Crohn's disease has been mapped to a large region spanning 18cM on chromosome 5q31 (Rioux et al., 2000 and 2001). Using dense maps of microsatellite markers and SNPs across the entire region, strong evidence of linkage disequilibrium (LD) was found. Having found evidence of LD, the authors developed an ultra-high-density SNP map and studied a denser collection of markers selected from this map. Multilocus analyses defined a single common risk haplotype characterised by multiple SNPs that were each independently associated using TDT. These SNPs were unique to the risk haplotype and essentially identical in their information content by virtue of being in nearly complete LD with one another. The equivalent properties of these SNPs make it impossible to identify the causal mutation within this region on the basis of genetic evidence alone.

### Causal Mutation

Mutations in the CNTNAP2 gene which are responsible for obesity or an associated disorder may be identified by comparing the sequences of the CNTNAP2 gene from patients presenting obesity or an associated disorder and control individuals. Based on the identified association of SNPs of CNTNAP2 and obesity or an associated disorder, the identified locus can be scanned for mutations. In a preferred embodiment, functional regions such as exons and splice sites, promoters and other regulatory regions of the CNTNAP2 gene are scanned for mutations. Preferably, patients presenting obesity or an associated disorder carry the mutation shown to be associated with obesity or an associated disorder and controls individuals do not carry the mutation or allele associated with obesity or an associated disorder. It might also be possible that patients presenting obesity or an associated disorder carry the mutation shown to be associated with obesity or an associated disorder with a higher frequency than controls individuals.

The method used to detect such mutations generally comprises the following steps: amplification of a region of the CNTNAP2 gene comprising a SNP or a group of SNPs associated with obesity or an associated disorder from DNA samples of the CNTNAP2 gene from patients presenting obesity or an associated disorder and control individuals; sequencing of the amplified region; comparison of DNA sequences of the CNTNAP2 gene from patients presenting obesity or an associated disorder and control individuals; determination of mutations specific to patients presenting obesity or an associated disorder.

Therefore, identification of a causal mutation in the CNTNAP2 gene can be carried out by the skilled person without undue experimentation by using well-known methods.

For example, the causal mutations have been identified in the following examples by using routine methods.

Hugot et al. (2001) applied a positional cloning strategy to identify gene variants with susceptibly to Crohn's disease in a region of chromosome 16 previously found to be linked to susceptibility to Crohn's disease. To refine the location of the potential sucecptibility locus 26 microsatellite markers were genotyped and tested for association to Crohn's disease using the transmission disequilibrium test. A borderline significant association was found between one allele of the microsatellite marker D16S136. Eleven additional SNPs were selected from surrounding regions and several SNPs showed significant association. SNP5-8 from this region were found to be present in a single exon of the NOD2/CARD15 gene and shown to be non-synonymous variants. This prompted the authors to sequence the complete coding sequence of this gene in 50 CD patients. Two additional non-synonymous mutations (SNP12 and SNP13) were found. SNP13 was most significant associated (p=6x10-6) using the pedigree transmission disequilibrium test. In another independent study, the same variant was found also by sequencing the coding region of this gene from 12 affected individuals compared to 4 controls (Ogura et al., 2001). The rare allele of SNP13 corresponded to a 1-bp insertion predicted to truncate the NOD2/CARD15 protein. This allele was also present in normal healthy individuals, albeit with significantly lower frequency as compared to the controls.

Similarly, Lesage et al. (2002) performed a mutational analyses of CARD 15 in 453 patients with CD, including 166 sporadic and 287 familial cases, 159 patients with ulcerative colitis (UC), and 103 healthy control subjects by systematic sequencing of the coding region. Of 67 sequence variations identified, 9 had an allele frequency >5% in patients with CD. Six of them were considered to be polymorphisms, and three (SNP12-R702W, SNPB-G908R, and SNP13-1007fs) were confirmed to be independently associated with susceptibility to CD. Also considered as potential disease-causing mutations (DCMs) were 27 rare additional mutations. The three main variants (R702W, G908R, and 1007fs) represented 32%, 18%, and 31%, respectively, of the total CD mutations, whereas the total of the 27 rare mutations represented 19% of DCMs. Altogether, 93% of the mutations were located in the distal third of the gene. No mutations were found to be associated with UC. In contrast, 50% of patients with CD carried at least one DCM, including 17% who had a double mutation.

### DRUG SCREENING

Novel targets and methods for the screening of drug candidates or leads are also provided. The methods include binding assays and/or functional assays, and may be performed in vitro, in cell systems, in animals, etc.

A particular object resides in a method of selecting compounds active on obesity or an associated disorder, said method comprising contacting in vitro a test compound with a CNTNAP2 gene or polypeptide according to the present invention and determining the ability of said test compound to bind said CNTNAP2 gene or polypeptide. Binding to said gene or polypeptide provides an indication as to the ability of the compound to modulate the activity of said target, and thus to affect a pathway leading to obesity or an associated disorder in a subject. In a preferred embodiment, the method comprises contacting in vitro a test compound with a CNTNAP2 polypeptide or a fragment thereof according to the present invention and determining the ability of said test compound to bind said CNTNAP2 polypeptide or fragment. The fragment preferably comprises a binding site of the CNTNAP2 polypeptide. Preferably, said CNTNAP2 gene or polypeptide or a fragment thereof is an altered or mutated CNTNAP2 gene or polypeptide or a fragment thereof comprising the alteration or mutation.

A particular object resides in a method of selecting compounds active on obesity or an associated disorder, said method comprising contacting in vitro a test compound with a CNTNAP2 polypeptide described herein or binding site-containing fragment thereof and determining the ability of said test compound to bind said CNTNAP2 polypeptide or fragment thereof. Preferably, said CNTNAP2 polypeptide or a fragment thereof is an altered or mutated CNTNAP2 polypeptide or a fragment thereof comprising the alteration or mutation.

In a further particular embodiment, the method comprises contacting a recombinant host cell expressing a CNTNAP2 polypeptide according to the present invention with a test compound, and determining the ability of said test compound to bind said CNTNAP2 and to modulate the activity of CNTNAP2 polypeptide. Preferably, said CNTNAP2 polypeptide or a fragment thereof is an altered or mutated CNTNAP2 polypeptide or a fragment thereof comprising the alteration or mutation.

The determination of binding may be performed by various techniques, such as by labeling of the test compound, by competition with a labeled reference ligand, etc.

A further object resides in a method of selecting compounds active on obesity or an associated disorder, said method comprising contacting in vitro a test compound with a CNTNAP2 polypeptide described herein and determining the ability of said test compound to modulate the activity of said CNTNAP2 polypeptide. Preferably, said CNTNAP2 polypeptide or a fragment thereof is an altered or mutated CNTNAP2 polypeptide or a fragment thereof comprising the alteration or mutation.

A further object resides in a method of selecting compounds active on obesity or an associated disorder, said method comprising contacting in vitro a test compound with a CNTNAP2 gene described herein and determining the ability of said test compound to modulate the expression of said CNTNAP2 gene. Preferably, said CNTNAP2 gene or a fragment thereof is an altered or mutated CNTNAP2 gene or a fragment thereof comprising the alteration or mutation.

Another embodiment relates to a method of screening, selecting or identifying active compounds, particularly compounds active on obesity or an associated disorder, the method comprising contacting a test compound with a recombinant host cell comprising a reporter construct, said reporter construct comprising a reporter gene under the control of a CNTNAP2 gene promoter, and selecting the test compounds that modulate (e.g. stimulate or reduce) expression of the reporter gene. Preferably, said CNTNAP2 gene promoter or a fragment thereof is an altered or mutated CNTNAP2 gene promoter or a fragment thereof comprising the alteration or mutation.

In a particular embodiment of the methods of screening, the modulation is an inhibition. In another particular embodiment of the methods of screening, the modulation is an activation.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

### PHARMACEUTICAL COMPOSITION, THERAPY

A further object is a pharmaceutical composition comprising (i) a CNTNAP2 polypeptide or a fragment thereof, a nucleic acid encoding a CNTNAP2 polypeptide or a fragment thereof, a vector or a recombinant host cell as described above and (ii) a pharmaceutically acceptable carrier or vehicle.

A method of treating or preventing obesity or an associated disorder in a subject, comprising administering to said subject a functional (e.g., wild-type) CNTNAP2 polypeptide or a nucleic acid encoding the same, is also described.

An other embodiment resides in a method of treating or preventing obesity or an associated disorder in a subject, the method comprising administering to said subject a compound that modulates, preferably that activates or mimics, expression or activity of a CNTNAP2 gene or protein described herein. Said compound can be an agonist or an antagonist of CNTNAP2, an antisense or a RNAi of CNTNAP2, an antibody or a fragment or a derivative thereof specific to a CNTNAP2 polypeptide. In a particular embodiment of the method, the modulation is an inhibition. In another particular embodiment of the method, the modulation is an activation.

The use of a functional CNTNAP2 polypeptide, a nucleic acid encoding the same, or a compound that modulates expression or activity of a CNTNAP2 gene or protein described herein, in the manufacture of a pharmaceutical composition for treating or preventing obesity or an associated disorder in a subject is also generally described. Said compound can be an agonist or an antagonist of CNTNAP2, an antisense or a RNAi of CNTNAP2, an antibody or a fragment or a derivative thereof specific to a CNTNAP2 polypeptide described herein. In a particular embodiment of the method, the modulation is an inhibition. In another particular embodiment of the method, the modulation is an activation.

The present disclosure demonstrates the correlation between obesity or an associated disorder and the CNTNAP2 gene locus. A novel target of therapeutic intervention is thus provided. Various approaches can be contemplated to restore or modulate the CNTNAP2 activity or function in a subject, particularly those carrying an altered CNTNAP2 gene locus. Supplying wild-type function to such subjects is expected to suppress phenotypic expression of obesity and associated disorders in a pathological cell or organism. The supply of such function can be accomplished through gene or protein therapy, or by administering compounds that modulate or mimic CNTNAP2 polypeptide activity (e.g., agonists as identified in the above screening assays).

The wild-type CNTNAP2 gene or a functional part thereof may be introduced into the cells of the subject in need thereof using a vector as described above. The vector may be a viral vector or a plasmid. The gene may also be introduced as naked DNA. The gene may be provided so as to integrate into the genome of the recipient host' cells, or to remain extra-chromosomal. Integration may occur randomly or at precisely defined sites, such as through homologous recombination. In particular, a functional copy of the CNTNAP2 gene may be inserted in replacement of an altered version in a cell, through homologous recombination. Further techniques include gene gun, liposome-mediated transfection, cationic lipid-mediated transfection, etc. Gene therapy may be accomplished by direct gene injection, or by administering ex vivo prepared genetically modified cells expressing a functional CNTNAP2 polypeptide.

Other molecules with CNTNAP2 activity (e.g., peptides, drugs, CNTNAP2 agonists, or organic compounds) may also be used to restore functional CNTNAP2 activity in a subject or to suppress the deleterious phenotype in a cell.

Restoration of functional CNTNAP2 gene function in a cell may be used to prevent the development of obesity or an associated disorder or to reduce progression of said diseases. Such a treatment may suppress the obesity-associated phenotype of a cell, particularly those cells carrying a deleterious allele.

Further aspects and advantages will be disclosed in the following experimental section, which should be regarded as illustrative and not limiting the scope of the present application.

### GENE, VECTORS, RECOMBINANT CELLS AND POLYPEPTIDES

A further aspect resides in novel products for use in diagnosis, therapy or screening. These products comprise nucleic acid molecules encoding a CNTNAP2 polypeptide or a fragment thereof, vectors comprising the same, recombinant host cells and expressed polypeptides.

More particularly, an altered or mutated CNTNAP2 gene or a fragment thereof comprising said alteration or mutation is concerned. Nucleic acid molecules encoding an altered or mutated CNTNAP2 polypeptide or a fragment thereof comprising said alteration or mutation are also concerned. Said alteration or mutation modifies the CNTNAP2 activity. The modified activity can be increased or decreased. A vector comprising an altered or mutated CNTNAP2 gene or a fragment thereof comprising said alteration or mutation or a nucleic acid molecule encoding an altered or mutated CNTNAP2 polypeptide or a fragment thereof comprising said alteration or mutation, recombinant host cells and expressed polypeptides, are further concerned.

A further object is a vector comprising a nucleic acid encoding a CNTNAP2 polypeptide described herein. The vector may be a cloning vector or, more preferably, an expression vector, i.e., a vector comprising regulatory sequences causing expression of a CNTNAP2 polypeptide from said vector in a competent host cell.

These vectors can be used to express a CNTNAP2 polypeptide in vitro, ex vivo or in vivo, to create transgenic or "Knock Out" non-human animals, to amplify the nucleic acids, to express antisense RNAs, etc.

The vectors described typically comprise a CNTNAP2 coding sequence according to the present invention operably linked to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and may provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

In this regard, a particular object resides in a recombinant virus encoding a CNTNAP2 polypeptide as defined above. The recombinant virus is preferably replication-defective, even more preferably selected from El- and/or E4-defective adenoviruses, Gag-, pol- and/or env-defective retroviruses and Rep- and/or Cap-defective AAVs. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

A further object resides in a recombinant host cell comprising a recombinant CNTNAP2 gene or a vector as defined above. Suitable host cells include, without limitation, prokaryotic cells (such as bacteria) and eukaryotic cells (such as yeast cells, mammalian cells, insect cells, plant cells, etc.). Specific examples include E. coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

A method for producing a recombinant host cell expressing a CNTNAP2 polypeptide described herein is also provided, said method comprising (i) introducing in vitro or ex vivo into a competent host cell a recombinant nucleic acid or a vector as described above, (ii) culturing in vitro or ex vivo the recombinant host cells obtained and (iii), optionally, selecting the cells which express the CNTNAP2 polypeptide.

Such recombinant host cells can be used for the production of CNTNAP2 polypeptides, as well as for screening of active molecules, as described below. Such cells may also be used as a model system to study obesity and associated disorders. These cells can be maintained in suitable culture media, such as DMEM, RPMI, HAM, etc., in any appropriate culture device (plate, flask, dish, tube, pouch, etc.).

### EXAMPLES

### 1. GenomeHIP platform to identify the chromosome 7 susceptibility gene

The GenomeHIP platform was applied to allow rapid identification of an obesity susceptibility gene.

Briefly, the technology consists of forming pairs from the DNA of related individuals. Each DNA is marked with a specific label allowing its identification. Hybrids are then formed between the two DNAs. A particular process (WO00/53802) is then applied that selects all fragments identical-by-descent (IBD) from the two DNAs in a multi step procedure. The remaining IBD enriched DNA is then scored against a BAC clone derived DNA microarray that allows the positioning of the IBD fraction on a chromosome.

The application of this process over many different families results in a matrix of IBD fractions for each pair from each family. Statistical analyses then calculate the minimal IBD regions that are shared between all families tested. Significant results (p-values) are evidence for linkage of the positive region with the trait of interest (here obesity). The linked interval can be delimited by the two most distant clones showing significant p-values.

In the present study, 164 families of German origin (178 independent sib-pairs) concordant for massive obesity (as defined by a body mass index > 90th%ile) were submitted to the GenomeHIP process. The resulting IBD enriched DNA fractions were then labelled with Cy5 fluorescent dyes and hybridised against a DNA array consisting of 2263 BAC clones covering the whole human genome with an average spacing of 1.2 Mega base pairs. Non-selected DNA labelled with Cy3 was used to normalize the signal values and compute ratios for each clone. Clustering of the ratio results was then performed to determine the IBD status for each clone and pair.

By applying this procedure, several BAC clones (BACA25ZB12, BACA2ZB02 and BACA20ZB03) spanning approximately 5.7 Mega bases in the region on chromosome 7 (bases 144 608 830 to 150 454 591) were identified, that showed significant evidence for linkage to obesity (p=1.20E-07).

Table 2: Linkage results for chromosome 7 in the CNTNAP2 locus: Indicated is the region correspondent to 3 BAC clones with evidence for linkage. The start and stop positions of the clones correspond to their genomic location based on NCBI Build34 sequence respective to the start of the chromosome (p-ter).

**Table 2**

| Human chromosome | Clones | Start | End | Proportion of informative pairs | p-value |
|---|---|---|---|---|---|
| 7 | BACA25ZB12 | 144 608 830 | 144 736 618 | 0.92 | 5.40E-05 |
| 7 | BACA2ZB02 | 147907717 | 148076897 | 0.88 | 1.20E-07 |
| 7 | BACA20ZB03 | 150 314 820 | 150 454 591 | 0.88 | 3.20E-05 |

### 2. Identification of an obesity susceptibility gene on chromosome 7

By screening the aforementioned 5.7 Megabases in the linked chromosomal region, we identified the contactin associated protein-like 2 (CNTNAP2) gene as a candidate for obesity and related phenotypes. This gene is indeed present in the critical interval, with evidence for linkage delimited by the clones outlined above.

CNTNAP2 gene encodes a predicted 1331-amino acid polypeptide for NP_054860 (mRNA NM_014141, 8107 bp) and spreads over 2304.258 kb of genomic sequence. The protein encoded by this gene is a member of the neurexin family which functions in the vertebrate nervous system as cell adhesion molecules and receptors. This protein, like other neurexin proteins, contains epidermal growth factor repeats and laminin G domains. In addition, it includes an F5/8 type C domain, discoidin/neuropilin- and fibrinogen-like domains, thrombospondin N-terminal-like domains and a putative PDZ binding site. This protein is localized at the juxtaparanodes of myelinated axons and associated with potassium channels.

CNTNAP2 precisely colocalized with Shaker-like potassium channels in the juxtaparanodal region (Poliak et al., 1999). CNTNAP2 specifically associated with Kv1.1 (KCNAl), Kv1.2 (KCNA2), and their Kv-beta-2 subunit (KCNA2). This association involved the C-terminal region of CNTNAP2. This lead the authors to suggest that CNTNAP2 may stabilize the localization of potassium channels in the juxtaparanodal region, and that CNTNAP2 family members may play a role in the local differentiation of the axon into distinct functional subdomains.

Significant linkage and association with KCNAB2 and KCNA1 was found in the same families as used in the present study (see patent application US 60/578,829) indicating that a set of proteins interacting with each other are jointly contributing to the development of obesity.

Voltage-gated potassium (Kv) channels represent the most complex class of voltage-gated ion channels from both functional and structural standpoints. Their diverse functions include regulating neurotransmitter release, heart rate, insulin secretion, neuronal excitability, epithelial electrolyte transport, smooth muscle contraction, and cell volume.

Recent investigations suggest that Kv channels are active participants in the regulation of beta-cell electrical activity and insulin secretion (MacDonald and Wheeler, 2003). Beta-cell Kv channels are targets of the G-protein coupled GLP-1 receptor and signals from glucose metabolism, pathways which could be physiologically relevant to the control of insulin secretion (MacDonald and Wheeler, 2003).

Examination of Kv1.3-deficient mice (Kv1.3(-/-)) revealed a previously unrecognized role for Kv1.3 in body weight regulation. Kv1.3(-/-) mice weighed significantly less than control littermates (Xu et al., 2003). Moreover, knockout mice were protected from diet-induced obesity and gained significantly less weight than littermate controls when placed on a high-fat diet.

McDaniel et al. (2001) reported an anorexic effect of K+ channel blockade by extracellular application of 4-aminopyridine (4-AP), a Kv-channel blocker, in mesenteric arterial smooth muscle (MASMC) and intestinal epithelial cells functionally expressing multiple Kv channel alpha- and beta-subunits including Kvbeta2.1 encoded by KCNAB2 in rats.

It has been demonstrated that the anorexic drugs, fenfluramine and dexenfluramine, in addition to inhibiting serotonin transporters (Baumann et al., 2000), decrease Kv channel activity in vascular smooth muscle cells (Hu et al., 1998, Michelakis et al., 1999; Wang et al., 1997). These observations suggest that the activity of Kv channels in MASMC may play an important role in the regulation of energy intake by controlling nutrient transportation.

Taken together, the linkage results provided in the present application, identifying the human CNTNAP2 gene in the critical interval of genetic alterations linked to obesity on chromosome 7, with its involvement in the interaction with voltage-gated potassium (Kv) channels, we conclude that alterations (e.g., mutations and/or polymorphisms) in the CNTNAP2 gene or its regulatory sequences may contribute to the development of human obesity and represent a novel target for diagnosis or therapeutic intervention.

### 3. Association study

The same families that have been used for the linkage study were also used to test for association between a specific phenotype (here obesity) in question and the genetic marker allele or haplotypes containing a specific marker allele using the transmission disequilibrium test (TDT). The TDT is a powerful association test as it is insensitive to population stratification problems in the tested sample. Briefly, the segregation of alleles from heterozygous parents to their affected offspring is tested. The portion of alleles transmitted to the affected offspring compared to the non-transmitted alleles is compared to the ratio expected under random distribution. A significant excess of allele transmission over the expected value is evidence for an association of the respective allele or haplotype with the studied obesity phenotype.

The results of this analysis show that certain alleles of the CNTNAP2 gene are positively associated with obesity and therefore increase the susceptibility to disease. In the tested population, for example, the allele 1 of SNP156 is correlated with obesity as determined by TDT (p-value = 0.000246). In contrast, the allele 2 of SNP156 is significantly under-transmitted to obese individuals showing that this allele helps protect from the disease. Other SNPs associated with obesity include SNP45, SNP48, SNP49, SNP50, SNP51, SNP53, SNP56, SNP60, SNP63, SNP65, SNP77, SNP94, SNP111, SNP112, SNP141, SNP142, and SNP163 as shown in the examples of the transmission of the alleles to obese patients in Table 3.

Examples of the transmission and non-transmission of the alleles to obese patients are given in Table 3.

**Table 3**

| SNP | Allele | Allele transmitted to obese individuals (N) | Allele not transmitted to obese individuals (N) | p- value |
|---|---|---|---|---|
| SNP45 | 1 | 121 | 92 | 0.01399 |
| SNP45 | 2 | 188 | 217 | 0.01399 |
| SNP48 | 1 | 119 | 154 | 0.03415 |
| SNP48 | 2 | 154 | 119 | 0.03415 |
| SNP49 | 1 | 144 | 107 | 0.019522 |
| SNP49 | 2 | 107 | 144 | 0.019522 |
| SNP50 | 1 | 176 | 138 | 0.031996 |
| SNP50 | 2 | 138 | 176 | 0.031996 |
| SNP51 | 1 | 166 | 194 | 0.02227 |
| SNP51 | 2 | 143 | 115 | 0.02227 |
| SNP53 | 1 | 131 | 167 | 0.037031 |
| SNP53 | 2 | 167 | 131 | 0.037031 |
| SNP56 | 1 | 175 | 208 | 0.007016 |
| SNP56 | 2 | 140 | 107 | 0.007016 |
| SNP60 | 1 | 208 | 166 | 0.029873 |
| SNP60 | 2 | 166 | 208 | 0.029873 |
| SNP63 | 1 | 167 | 208 | 0.03424 |
| SNP63 | 2 | 208 | 167 | 0.03424 |
| SNP65 | 1 | 198 | 160 | 0.044605 |
| SNP65 | 2 | 160 | 198 | 0.044605 |
| SNP77 | 1 | 204 | 178 | 0.03646 |
| SNP77 | 2 | 117 | 143 | 0.03646 |
| SNP94 | 1 | 140 | 109 | 0.049467 |
| SNP94 | 2 | 109 | 140 | 0.049467 |
| SNP111 | 1 | 156 | 110 | 0.004796 |
| SNP111 | 2 | 110 | 156 | 0.004796 |
| SNP112 | 1 | 118 | 87 | 0.030377 |
| SNP112 | 2 | 87 | 118 | 0.030377 |
| SNP141 | 1 | 191 | 152 | 0.035222 |
| SNP141 | 2 | 152 | 191 | 0.035222 |
| SNP142 | 1 | 184 | 139 | 0.012284 |
| SNP142 | 2 | 139 | 184 | 0.012284 |
| SNP156 | 1 | 118 | 68 | 0.000246 |
| SNP156 | 2 | 68 | 118 | 0.000246 |
| SNP163 | 1 | 262 | 240 | 0.02604 |
| SNP163 | 2 | 50 | 72 | 0.02604 |

In addition, haplotypes were constructed for all the SNPs included in Table 3 apart from SNP94 plus SNP11, SNP14, SNP40, SNPSNP47, SNP65, SNP113, SNP116, SNP142 and SNP161 to identify the phase for all SNPs.

The results of this analysis in the tested population showed that certain haplotypes are strongly associated with obesity, while certain haplotypes are preferentially not transmitted to obese patients. An example for a haplotype that is preferentially transmitted to obese patients is the haplotype 1-2-1-1 for SNP50-SNP56-SNP113-SNP156, p = 4.946⁻⁰⁶. An example for a haplotype that is preferentially not transmitted to obese patients is the haplotype 2-1-1-2 for SNP50-SNP56-SNP113-SNP156, p = 0.0007862.

Examples of haplotypes with preferential transmission and non-transmission to obese patients are given in Table 4.

**Table 4**

| SNPs used to construct haplotype | Alleles composing haplotype | Frequency of haplotype transmitted to obese patients | Frequency of haplotype not transmitted to obese patients | p-value |
|---|---|---|---|---|
| 40-50-156 | 1-1-1 | 0.2631 | 0.1273 | 2.12e-05 |
| 40-50-156 | 1-2-2 | 0.05078 | 0.1181 | 0.001761 |
| 50 113 156 | 1-1-1 | 0.2792 | 0.1342 | 1.312e-05 |
| 50113156 | 2-1-2 | 0.05991 | 0.1241 | 0.004561 |
| 50-116-156 | 1-2-1 | 0.1732 | 0.06897 | 7.213e-05 |
| 50-56-113-156 | 1-2-1-1 | 0.2481 | 0.1068 | 4.946e-06 |
| 50-56-113-156 | 2-1-1-2 | 0.03738 | 0.1069 | 0.0007862 |
| 40-50-56-156 | 1-1-2-1 | 0.2211 | 0.0916 | 7.59e-06 |
| 40-50-56-156 | 1-2-1-2 | 0.0341 | 0.09947 | 0.001165 |
| 40-56-113-156 | 1-1-1-2 | 0.02471 | 0.07988 | 0.002167 |
| 40-56-113-156 | 1-2-1-1 | 0.2581 | 0.1238 | 4.269e-05 |
| 40-50-113-156 | 1-1-1-1 | 0.2135 | 0.08298 | 8.213e-06 |
| 45-48-156 | 1-2-1 | 0.2398 | 0.1192 | 0.000123 |
| 45-48-156 | 2-1-2 | 0.0832 | 0.1322 | 0.02454 |
| 45-14-156 | 1-1-1 | 0.1341 | 0.05972 | 0.001942 |
| 45-14-156 | 1-2-1 | 0.2103 | 0.1429 | 0.02729 |
| 45-14-156 | 1-2-2 | 0.02364 | 0.06296 | 0.01976 |
| 45-14-156 | 2-1-2 | 0.02232 | 0.05554 | 0.02821 |
| 45-14-156 | 1-1-1 | 0.1782 | 0.08875 | 0.0007682 |
| 45-14-156 | 1-2-2 | 0.01635 | 0.06292 | 0.003773 |
| 45-14-156 | 2-1-2 | 0.02617 | 0.06711 | 0.01712 |
| 47-48-156 | 1-1-2 | 0.0903 | 0.1474 | 0.01483 |
| 47-48-156 | 2-2-1 | 0.2511 | 0.1312 | 0.0002089 |
| 47-11-156 | 1-2-2 | 6.54e-11 | 0.08162 | 2.907e-07 |
| 47-11-156 | 2-1-1 | 0.2521 | 0.181 | 0.006931 |
| 48-65-156 | 1-1-2 | 0.04111 | 0.1012 | 0.004426 |
| 48-65-156 | 2-2-1 | 0.174 | 0.1014 | 0.009455 |
| 48-11-156 | 1-2-2 | 0.004645 | 0.08581 | 4.385e-07 |
| 48-11-156 | 2-1-1 | 0.2227 | 0.1416 | 0.005923 |
| 49-141-156 | 1-1-1 | 0.1592 | 0.0509 | 0.0001743 |
| 49-141-156 | 2-1-2 | 0.02592 | 0.05667 | 0.04417 |
| 50-51-156 | 1-2-1 | 0.3344 | 0.1934 | 0.0001126 |
| 50-51-156 | 2-1-2 | 0.0788 | 0.1529 | 0.002454 |
| 50-60-111 | 1-1-1 | 0.1549 | 0.08485 | 0.00394 |
| 50-60-111 | 2-1-2 | 0.05278 | 0.1177 | 0.007073 |
| 50-60-156 | 1-1-1 | 0.1799 | 0.0997 | 0.002196 |
| 50-60-156 | 1-2-1 | 0.163 | 0.1067 | 0.04082 |
| 50-60-156 | 2-1-2 | 0.05528 | 0.1003 | 0.02889 |
| 50-60-156 | 2-2-1 | 0.2105 | 0.2953 | 0.01913 |
| 50-63-156 | 1-1-1 | 0.1877 | 0.1 | 0.002533 |
| 50-63-156 | 1-2-1 | 0.1581 | 0.1081 | 0.03409 |
| 50-63-156 | 2-1-1 | 0.2673 | 0.3598 | 0.02274 |
| 50-63-156 | 2-2-2 | 0.04493 | 0.08649 | 0.02845 |
| 50-65-156 | 1-1-1 | 0.1605 | 0.1065 | 0.02477 |
| 50-65-156 | 1-2-1 | 0.1861 | 0.09773 | 0.002379 |
| 50-65-156 | 2-1-2 | 0.03714 | 0.08847 | 0.00934 |
| 50-65-156 | 2-2-1 | 0.2738 | 0.3678 | 0.02505 |
| 50-112-141 | 1-1-1 | 0.125 | 0.06503 | 0.01111 |
| 50-112-141 | 2-2-1 | 0.01719 | 0.08347 | 0.0005085 |
| 50-141-156 | 1-1-1 | 0.142 | 0.05543 | 0.0004759 |
| 50-141-156 | 1-2-1 | 0.202 | 0.153 | 0.05193 |
| 50-141-156 | 2-1-2 | 0.02591 | 0.057 | 0.03197 |
| 50-142-156 | 1-1-1 | 0.187 | 0.07765 | 5.547e-05 |
| 50-142-156 | 2-1-2 | 0.03671 | 0.0747 | 0.03206 |
| 50-156-161 | 1-1-1 | 0.1628 | 0.0897 | 0.005409 |
| 50-156-161 | 1-1-2 | 0.1796 | 0.1166 | 0.01194 |
| 50-156-161 | 2-2-2 | 0.06924 | 0.1473 | 0.001339 |
| 50-156-163 | 1-1-1 | 0.3015 | 0.1645 | 5.931e-05 |
| 50-156-163 | 1-2-2 | 0.01869 | 0.02398 | 0.02484 |
| 50-156-163 | 2-2-2 | 0.02621 | 0.06409 | 0.01847 |
| 53-60-156 | 1-1-1 | 0.3509 | 0.2698 | 0.03822 |
| 53-60-156 | 1-2-1 | 0.2179 | 0.3078 | 0.01797 |
| 53-60-156 | 2-2-1 | 0.1618 | 0.09989 | 0.03112 |
| 53-111-156 | 1-2-2 | 0.006907 | 0.0784 | 5.113e-06 |
| 53-111-156 | 2-1-1 | 0.2199 | 0.1587 | 0.03123 |
| 53-112-156 | 1-2-2 | 0.01046 | 0.05976 | 0.0004787 |
| 53-112-156 | 2-1-1 | 0.2304 | 0.1548 | 0.0132 |
| 60-77-111 | 1-1-1 | 0.2695 | 0.1949 | 0.02817 |
| 60-77-111 | 1-1-2 | 0.04658 | 0.1062 | 0.02026 |
| 60-77-111 | 2-2-1 | 0.09236 | 0.1544 | 0.02134 |
| 60-77-111 | 2-2-2 | 0.03902 | 0.09576 | 0.00513 |
| 60-77-142 | 1-1-1 | 0.1677 | 0.09388 | 0.007573 |
| 60-77-142 | 2-2-1 | 0.05143 | 0.08728 | 0.09579 |
| 60-77-142 | 2-2-2 | 0.07952 | 0.1637 | 0.001891 |
| 60-111-156 | 1-1-1 | 0.3934 | 0.2572 | 0.0003834 |
| 60-111-156 | 1-2-2 | 0.004562 | 0.05028 | 0.0004276 |
| 60-111-156 | 2-2-2 | 0.006349 | 0.03684 | 0.00462 |
| 63-77-111 | 1-2-2 | 0.04488 | 0.1126 | 0.002868 |
| 63-77-111 | 2-1-2 | 0.03732 | 0.09388 | 0.01354 |
| 63-111-156 | 1-2-2 | 0.005687 | 0.04263 | 0.01254 |
| 63-111-156 | 2-1-1 | 0.3211 | 0.227 | 0.006942 |
| 63-111-156 | 2-2-2 | 0.005947 | 0.04458 | 3.394e-05 |
| 77-111-156 | 1-1-1 | 0.4369 | 0.3339 | 0.005773 |
| 77-111-156 | 1-2-2 | 0.008825 | 0.03817 | 0.01577 |
| 77-111-156 | 2-2-2 | 0.004723 | 0.05016 | 0.0002808 |
| 111-142-156 | 1-1-1 | 0.3236 | 0.2381 | 0.01006 |
| 111-142-156 | 2-1-2 | 0.007099 | 0.04145 | 0.01948 |
| 111-142-156 | 2-2-2 | 0.006951 | 0.04059 | 0.0008523 |
| 111-156-161 | 1-2-1 | 0.02353 | 0.01563 | 0.05112 |
| 111-156-161 | 2-2-1 | 0.01273 | 0.008456 | 0.05269 |
| 111-156-161 | 2-2-2 | 0.007709 | 0.07174 | 2.86e-05 |
| 111-156-163 | 1-1-1 | 0.5671 | 0.4723 | 0.01049 |
| 111-156-163 | 2-2-1 | 0.005063 | 0.05258 | 0.0006018 |
| 111-156-163 | 2-2-2 | 0.01374 | 0.02757 | 0.002219 |

### REFERENCES

Baumann MH, Ayestas MA, Dersch CM et al. (2000) Serotonin transporters, serotonin release, and the mechanism of fenfluramine neurotoxicity. Ann N Y Acad Sci 914:172-86
Chagnon YC, Rankinen T, Snyder EE et al. (2003) The human obesity gene map: The 2002 update. Obes Res. 11(3):313-367.
Hebebrand J, Hescker H, Himmelmann GW, Schäfer H, Remschmidt H (1994) Percentiles for the body mass index based on data of the German national nutrition survey and a review of relevant factors with an influence on body weight. Aktuelle Ernaehrungsmedizin 19: 259-265.
Hebebrand J, Himmelmann GW, Hescker H, Schäfer H, Remschmidt H (1996) Use of percentiles for the body mass index in anorexia nervosa : diagnostic, epidemiological and therapeutic considerations. Int J Eat Dis 19: 359-369.
Hebebrand J, Hinney A, Roth H et al. (1998) Genetische Aspekte der Adipositas. In:J Wechsler (ed) Adipositas. Ex Libris Roche. Blackwell Verlag: 105-118.
Hinney A, Schmidt A, Nottebom K et al. (1999) Several mutations in the melanocortin-4 receptor gene including a nonsense and a frameshift mutation associated with dominantly inherited obesity in humans. J. Clin Endocrinol Metab. Apr; 84(4):1483-1486.
Hu S, Wang S, Gibson J et al (1998) Inhibition of delayed rectifier K+ channels by dexfenfluramine (Redux). J Pharmacol Exp Ther. 287(2):480-6.
Hugot JP, Chamaillard M, Zouali H et al. (2001) Association of NOD2 leucine-rich repeat variants with susceptibility to Crohn's disease. Nature 411(6837):599-603.
Huszar D, Lynch CA, Fairchild-Huntress V et al. (1997) Targeted disruption of the melanocortin-4 receptor results in obesity in mice. Cell 88:131-141.
Lander E and Kruglyak L (1995) Genetic dissection of complex traits: guidelines for interpreting and reporting linkage results. Nat Genet, 11(3):241-247.
Lesage S, Zouali H, Cezard Jpet al. (2002) CARD15/NOD2 mutational analysis and genotype-phenotype correlation in 612 patients with inflammatory bowel disease. Am J Hum Genet. 70(4):845-857.
MacDonald PE and Wheeler MB (2003) Voltage-ependent K+ channels in pancreatic beta cells: Role, regulation and potential as therapeutic target. Diabetologia 46:1046-1062.
McDaniel SS, Platoshyn O, Yu Y et al. (2001) Anorexic effect of K+ channel blockade in mesenteric arterial smooth muscle and intestinal epithelial cells. J Appl Physiol. 91(5):2322-2333.
Michelakis ED, Weir EK, Nelson DP et al. (1999) Dexfenfluramine elevates systemic blood pressure by inhibiting potassium currents in vascular smooth muscle cells. J Pharmacol Exp Ther. 291(3):1143-9.
Ogura Y, Bonen DK, Inohara N (2001) A framshift mutation in NOD2 associated with susceptibility to Crohn's disease. Nature 411(6837):603-606.
Poliak S, Gollan L, Martinez R et al. (1999) Caspr2, a new member of the neurexin superfamily, is localized at the juxtaparanodes of myelinated axons and associates with K+ channels. Neuron 24(4):1037-1047.
Rioux JD, Daly MJ, Silverberg MS et al. (2001) Genetic variation in the 5q31 cytokine gene cluster confers susceptibility to Crohn disease. Nat Genet 29(2): 223-228.
Rioux JD, Silverberg MS, Daly MJ (2000) Genomewide search in Canadian families with inflammatory bowel disease reveals two novel susceptibility loci. Am J Hum Genet 66(6):1863-1870.
Schneider R (1996) Relevanz und Kosten der Adipositas in Deutschland. Ernährungs-Umschau 43:369-374.
Vaisse C, Clément K, Guy-Grand B et al. (1998) A frameshift mutation in human MC4R is associated with a dominant form of obesity. Nat Genet 20:113-114.
Wang J, Juhaszova M, Rubin LJ et al. (1997) Hypoxia inhibits gene expression of voltage-gated K+ channel alpha subunits in pulmonary artery smooth muscle cells. J Clin Invest. 100(9):2347-53.
WHO (1998) Preventing and managing the global epidemic. WHO, Geneva.
Yeo GS, Farooqi IS, Aminian S et al. (1998) A frameshift mutation in MC4R associated with dominantly inherited human obesity. Nat Genet 20:111-112.
Wolf AM and Colditz GA (1996) Social and economic effects of body weight in the United States. Am J Clin Nutr 63(3 Suppl):466S-469S.
Xu J, Koni PA, Wang P et al. (2003) The voltage-gated potassium channel Kv1.3 regulates energy homeostasis and body weight. Hum Mol Genet 12(5):551-9.
Zhang Y, Proenca R, Maffei M, Barone M, Leopold L, Friedman JM. (1994) Positional cloning of the mouse obese gene and its human homologue. Nature 372: 425-432.

## Claims

1. An *in vitro* method of detecting the presence of or predisposition to obesity in a subject, the method comprising detecting the presence of an alteration in the sequence of the CNTNAP2 gene, in a sample from said subject, wherein the alteration is a mutation of a base, an insertion or a deletion of one or more bases.

2. The method of claim 1, wherein the presence of an alteration in the CNTNAP2 gene locus is detected by sequencing, selective hybridisation and/or selective amplification.

3. The method of any one of claims 1 or 2, wherein said alteration is one or several SNP(s) or an haplotype ofSNPs associated with obesity.

4. The method of claim 3, wherein said haplotype associated with obesity comprises several SNPs selected in the group consisting of SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 and SNP163, as identified below:
| Position in chrom 7 based on NCBI Build 34 | SNP identity | dbSNP reference | Allele 1 | Allele 2 | Position in locus and type of amino acid change | SEQ ID No |
|---|---|---|---|---|---|---|
| 143467490 | 11 | rs1635079 | A | G | 5' of CNTNAP2 | 3 |
| 143543795 | 14 | rs886469 | C | T | 5' ofCNTNAP2 | 4 |
| 145272638 | 40 | rs12703780 | A | G | intron | 5 |
| 145367704 | 45 | rs1405109 | C | T | intron | 6 |
| 145399533 | 47 | rs7806058 | A | G | intron | 7 |
| 145424265 | 48 | rs7788551 | C | T | intron | 8 |
| 145446406 | 49 | rs7809716 | A | G | intron | 9 |
| 145456858 | 50 | rs6464757 | C | T | intron | 10 |
| 145484824 | 51 | rs1639481 | A | G | intron | 11 |
| 145519990 | 53 | rs7784672 | A | G | intron | 12 |
| 145568121 | 56 | rs700273 | G | T | intron | 13 |
| 145654078 | 60 | rs12535870 | A | G | intron | 14 |
| 145709388 | 63 | rs1155384 | C | T | intron | 15 |
| 145734063 | 65 | rs7789802 | A | C | intron | 16 |
| 146043201 | 77 | rs13221567 | A | C | intron | 17 |
| 146312130 | 94 | Not available | C | G | intron | 18 |
| 146603365 | 111 | rs851840 | C | G | intron | 19 |
| 146609747 | 112 | rs2888493 | A | G | intron | 20 |
| 146617641 | 113 | rs11764315 | A | C | intron | 21 |
| 146668312 | 116 | rs989242 | A | G | intron | 22 |
| 147225657 | 141 | rs2972125 | C | T | intron | 23 |
| 147254373 | 142 | rs963314 | A | G | intron | 24 |
| 147436213 | 156 | rs10278502 | C | T | intron | 25 |
| 147503860 | 161 | rs2270069 | C | T | intron | 26 |
| 147544244 | 163 | rs2717775 | C | G | 3' of CNTNAP2 | 27 |

5. The method of claim 3, wherein said SNP associated with obesity is selected from the group consisting of SNP45, SNP48, SNP49, SNP50, SNP51, SNP53, SNP56, SNP60, SNP63, SNP65, SNP77, SNP94, SNP111, SNP112, SNP141, SNP142, SNP156, and SNP161.

6. The method of claim 5, wherein the alteration in the sequence of the CNTNAP2 gene is allele 1 (nucleotide C) of SNP156.

7. The method of claim 4, wherein the alteration in the sequence of the CNTNAP2 gene is haplotype consisting of alleles 1-2-1-1 for SNP50-SNP56-SNP113-SNP156.

## Patentansprüche

1. *In vitro* Verfahren zur Detektion der Anwesenheit von oder der Prädisposition für Adipositas in einem Subjekt, wobei das Verfahren den Schritt umfasst, die Anwesenheit einer Veränderung in der Sequenz des CNTNAP2 Gens in einer Probe des genannten Subjekts zu detektieren, wobei die Veränderung eine Mutation einer Base, eine Insertion oder eine Deletion einer oder mehrerer Basen ist.

2. Verfahren nach Anspruch 1, wobei die Anwesenheit einer Veränderung im CNTNAP2 Genlokus durch Sequenzierung, selektive Hybridisierung und/oder selektive Amplifikation detektiert wird.

3. Verfahren nach einem der Ansprüche 1 oder 2, wobei die genannte Veränderung, die mit Adipositas assoziiert ist, ein oder mehrere SNP(s) oder ein Haplotyp von SNPs ist.

4. Verfahren nach Anspruch 3, wobei der genannte Haplotyp, der mit Adipositas assoziiert ist, mehrere SNPs umfasst, wobei die SNPs ausgewählt sind aus SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 und SNP163, wie unten identifiziert:
| Position auf Chromosom 7, basierend auf NCBI Build 34 | SNP Identität | dbSNP Referenz | Allel 1 | Allel 2 | Position in Lokus und Art des Aminosäure-Austauschs | SEQ ID Nr. |
|---|---|---|---|---|---|---|
| 143467490 | 11 | rs1635079 | A | G | 5' von CNTNAP2 | 3 |
| 143543795 | 14 | rs886469 | C | T | 5' von CNTNAP2 | 4 |
| 145272638 | 40 | rs12703780 | A | G | intron | 5 |
| 145367704 | 45 | rs1405109 | C | T | intron | 6 |
| 145399533 | 47 | rs7806058 | A | G | intron | 7 |
| 145424265 | 48 | rs7788551 | C | T | intron | 8 |
| 145446406 | 49 | rs7809716 | A | G | intron | 9 |
| 145456858 | 50 | rs6464757 | C | T | intron | 10 |
| 145484824 | 51 | rs1639481 | A | G | intron | 11 |
| 145519990 | 53 | rs7784672 | A | G | intron | 12 |
| 145568121 | 56 | rs700273 | G | T | intron | 13 |
| 145654078 | 60 | rs12535870 | A | G | intron | 14 |
| 145709388 | 63 | rs1155384 | C | T | intron | 15 |
| 145734063 | 65 | rs7789802 | A | C | intron | 16 |
| 146043201 | 77 | rs13221567 | A | C | intron | 17 |
| 146312130 | 94 | Nicht verfügbar | C | G | intron | 18 |
| 146603365 | 111 | rs851840 | C | G | intron | 19 |
| 146609747 | 112 | rs2888493 | A | G | intron | 20 |
| 146617641 | 113 | rs11764315 | A | C | intron | 21 |
| 146668312 | 116 | rs989242 | A | G | intron | 22 |
| 147225657 | 141 | rs2972125 | C | T | intron | 23 |
| 147254373 | 142 | rs963314 | A | G | intron | 24 |
| 147436213 | 156 | rs10278502 | C | T | intron | 25 |
| 147503860 | 161 | rs2270069 | C | T | intron | 26 |
| 147544244 | 163 | rs2717775 | C | G | 3' von CNTNAP2 | 27 |

5. Verfahren nach Anspruch 3, wobei das genannte SNPs, die mit Adipositas assoziiert ist, ausgewählt ist aus SNP45, SNP48, SNP49, SNP50, SNP51, SNP53, SNP56, SNP60, SNP63, SNP65, SNP77, SNP94, SNP111, SNP112, SNP141, SNP142, SNP156 und SNP161.

6. Verfahren nach Anspruch 5, wobei die Veränderung in der Sequenz des CNTNAP2 Gens das Allel 1 (Nukleotid C) von SNP156 ist.

7. Verfahren nach Anspruch 4, wobei die Veränderung in der Sequenz des CNTNAP2 Gens ein Haplotyp ist, der aus den Allelen 1-2-1-1 für SNP50-SNP56-SNP113-SNP156 besteht.

## Revendications

1. Procédé *in vitro* de détection de la présence de ou d'une prédisposition à l'obésité chez un sujet, le procédé comprenant la détection de la présence d'une altération dans la séquence du gène CNTNAP2, dans un échantillon provenant dudit sujet, dans lequel l'altération est une mutation d'une base, une insertion ou une délétion d'une ou plusieurs bases.

2. Procédé selon la revendication 1, dans lequel la présence d'une altération dans le locus du gène CNTNAP2 est détectée par séquençage, hybridation sélective et/ou amplification sélective.

3. Procédé selon l'une quelconque des revendications 1 ou 2, dans lequel ladite altération est un ou plusieurs SNP(s) ou un haplotype de SNPs associé à l'obésité.

4. Procédé selon la revendication 3, dans lequel ledit haplotype associé à l'obésité comprend plusieurs SNPs choisis dans le groupe constitué du SNP11, SNP14, SNP40, SNP45, SNP47, SNP48, SNP49, SNP50, SNP51, SNP53, SNP56, SNP60, SNP63, SNP65, SNP77, SNP94, SNP111, SNP112, SNP113, SNP116, SNP141, SNP142, SNP156, SNP161 et SNP163, comme identifiés ci-dessous :
| Position sur chrom 7 basée sur la construction 34 NCBI | Identité de SNP | Référence SNP db | Allèle 1 | Allèle 2 | Position dans le locus et type de changement d'acide aminé | SEQ ID No |
|---|---|---|---|---|---|---|
| 143467490 | 11 | rs1635079 | A | G | 5' de CNTNAP2 | 3 |
| 143543795 | 14 | rs886469 | C | T | 5' de CNTNAP2 | 4 |
| 145272638 | 40 | rs12703780 | A | G | intron | 5 |
| 145367704 | 45 | rs1405109 | C | T | intron | 6 |
| 145399533 | 47 | rs7806058 | A | G | intron | 7 |
| 145424265 | 48 | rs7788551 | C | T | intron | 8 |
| 145446406 | 49 | rs7809716 | A | G | intron | 9 |
| 145456858 | 50 | rs6464757 | C | T | intron | 10 |
| 145484824 | 51 | rs1639481 | A | G | intron | 11 |
| 145519990 | 53 | rs7784672 | A | G | intron | 12 |
| 145568121 | 56 | rs700273 | G | T | intron | 13 |
| 145654078 | 60 | rs12535870 | A | G | intron | 14 |
| 145709388 | 63 | rs1155384 | C | T | intron | 15 |
| 145734063 | 65 | rs7789802 | A | C | intron | 16 |
| 146043201 | 77 | rs13221567 | A | C | intron | 17 |
| 146312130 | 94 | Non disponible | C | G | intron | 18 |
| 146603365 | 111 | rs851840 | C | G | intron | 19 |
| 146609747 | 112 | rs2888493 | A | G | intron | 20 |
| 146617641 | 113 | rs11764315 | A | C | intron | 21 |
| 146668312 | 116 | rs989242 | A | G | intron | 22 |
| 147225657 | 141 | rs2972125 | C | T | intron | 23 |
| 147254373 | 142 | rs963314 | A | G | intron | 24 |
| 147436213 | 156 | rs10278502 | C | T | intron | 25 |
| 147503860 | 161 | rs2270069 | C | T | intron | 26 |
| 147544244 | 163 | rs2717775 | C | G | 3'de CNTNAP2 | 27 |

5. Procédé selon la revendication 3, dans lequel ledit SNP associé à l'obésité est choisi dans le groupe constitué du SNP45, SNP48, SNP49, SNP50, SNP51, SNP53, SNP56, SNP60, SNP63, SNP65, SNP77, SNP94, SNP111, SNP112, SNP141, SNP142, SNP156 et SNP161.

6. Procédé selon la revendication 5, dans lequel l'altération dans la séquence du gène CNTNAP2 est l'allèle 1 (nucléotide C) du SNP156.

7. Procédé selon la revendication 4, dans lequel l'altération dans la séquence du gène CNTNAP2 est l'haplotype constitué des allèles 1-2-1-1 pour SNP50-SNP56-SNP113-SNP156.
